# NEW EUROPEAN PATENT SPECIFICATION

(11) **EP 1 814 590 B2**
(45) Date of publication and mention of the opposition decision: **11.12.2013**
(45) Mention of the grant of the patent: 08.04.2009
(21) Application number: 05820773.9
(22) Date of filing: 01.11.2005
(51) Int. Cl.: A61K 38/22, A61K 45/06

(54) **TREATMENT OF OBESITY AND RELATED DISORDERS**
BEHANDLUNG VON FETTSUCHT UND VERBUNDENEN ERKRANKUNGEN
TRAITEMENT CONTRE L'OBESITE AINSI QUE LES MALADIES ASSOCIEES A L'OBESITE

(30) Priority: 01.11.2004 US 624357 P
(43) Date of publication of application: 08.08.2007
(60) Divisional application: 09003342.4
(73) Proprietor: AMYLIN PHARMACEUTICALS, INC., San Diego, CA 92121 (US)
(72) Inventor: ROTH, Jonathan, San Diego, California 92121 (US); ANDERSON, Christen, M., Encinitas, California 92024 (US); BARON, Alain, San Diego, California 92121 (US)
(74) Representative: HOFFMANN EITLE
(86) International application number: PCT/US2005/039686
(87) International publication number: WO 2006/052608

(56) References cited:
- WO-A-00/66629
- WO-A-98/30231
- WO-A-03/000663
- WO-A-03/045920
- WO-A-03/047568
- WO-A-03/057235
- WO-A-2004/009015
- WO-A-2004/056324
- WO-A1-00/25806
- WO-A1-96/31526
- WO-A1-98/22128
- WO-A1-98/55144
- US-A1- 2003 073 728
- US-A1- 2003 220 255
- US-A1- 2004 116 657
- US-A1- 2004 132 752
- US-B1- 6 313 298
- US-B2- 6 458 924
- GUTZWILLER JEAN-PIERRE ET AL: "Interaction between GLP-1 and CCK-33 in inhibiting food intake and appetite in men." AMERICAN JOURNAL OF PHYSIOLOGY. REGULATORY, INTEGRATIVE AND COMPARATIVE PHYSIOLOGY. SEP 2004, vol. 287, no. 3, September 2004 (2004-09), pages R562-R567, XP009070447 ISSN: 0363-6119
- MATSON CLAIRE A ET AL: "Synergy between leptin and cholecystokinin (CCK) to control daily caloric intake" PEPTIDES (TARRYTOWN), vol. 18, no. 8, 1997, pages 1275-1278, XP009070457 ISSN: 0196-9781
- MATSON C A ET AL: "Cholecystokinin and leptin act synergistically to reduce body weight." AMERICAN JOURNAL OF PHYSIOLOGY. REGULATORY, INTEGRATIVE AND COMPARATIVE PHYSIOLOGY. APR 2000, vol. 278, no. 4, April 2000 (2000-04), pages R882-R890, XP002393563 ISSN: 0363-6119
- EIDEN ET AL.: 'Salmon calcitonin' JOURNAL OF PHYSIOLOGY vol. 541,3, June 2002, pages 1041 - 1048
- RUSHING ET AL.: 'Amylin: A Novel Action in the Brain to Reduce Body Weight' ENDOCRINOLOGY vol. 141, no. 2, February 2000, pages 850 - 853
- ARNELO ET AL.: 'Effects of long-term infusion of anorexic concentrations of islet amyloid polypeptide on neurotransmitters and neuropeptides in rat brain' BRAIN RESEARCH vol. 887, 2000, pages 391 - 398
- GRABLER ET AL.: 'Chronic infusion of the amylin antagonist AC 187' PHYSIOLOGY & BEHAVIOR vol. 81, 05 February 2004, pages 481 - 488
- MORLEY ET AL.: 'Modulation of food intake by peripherally administered' AM J PHYSIOL vol. 267, July 1994, pages 178 - 184
- HEYMSFIELD ET AL.: 'Recombinant Leptin for Weight Loss in Obese and Lean Adults' JAMA vol. 282, no. 16, October 1999, pages 1568 - 1575
- CHICUREL: 'Whatever happened to leptin?' NATURE vol. 404, 06 April 2000, pages 538 - 540
- BAYS: 'Current and Investigational Antiobesity Agents' OBESITY RESEARCH vol. 12, no. 8, 08 August 2004, pages 1197 - 1211
- TENENBAUM: 'Leptin's Legacy' HHMI BULLETIN March 2003, pages 25 - 27
- CONSIDINE ET AL.: 'Serum Immunoreactive-Leptin Concentrations in Normal-Weight' THE NEW ENGLAND JOURNAL OF MEDICINE vol. 334, 01 February 1996, pages 292 - 295
- BERTHOUD: 'The Caudal Brainstem and the Control of Food Intake' HANDBOOK OF BEHAVIORAL NEUROBIOLOGY vol. 14, no. 2ND ED, 2004, pages 195 - 240
- LEHNINGER: 'hormonal regulation and integration of mammalian metabolism' PRINCIPLES OF BIOCHEMISTRY vol. 4TH ED, no. CHAP23, April 2004,
- Excerpts from Amgen's US SEC form 10-K for the fiscal years 1999 & 2001
- Excerpt from forbes.com (http://www.forbes.com/forbes/2002/1125/273 .
- OSTLUND ET AL.: 'Relation between plasma leptin concentration and body fat,' JOURNAL OF CLINICAL ENDOCRINOLOGY AND METABOLISM vol. 81, no. 11, 01 May 1996, pages 3909 - 3913
- SHAMSUZZAMAN ET AL.: 'Independent Association Between Plasma Leptin and C-Reactive' CIRCULATION vol. 109, 26 April 2004, pages 2181 - 2185

## Description

### FIELD OF THE INVENTION

The present invention relates to the medical field and in particular to the field of health, diet and nutrition. The invention relates to the use of anti-obesity agents.

### BACKGROUND

Obesity and its associated disorders are common and very serious public health problems in the United States and throughout the world. Upper body obesity is the strongest risk factor known for type 2 diabetes mellitus and is a strong risk factor for cardiovascular disease. Obesity is a recognized risk factor for hypertension, atherosclerosis, congestive heart failure, stroke, gallbladder disease, osteoarthritis, sleep apnea, reproductive disorders such as polycystic ovarian syndrome, cancers of the breast, prostate, and colon, and increased incidence of complications of general anesthesia (*see, e.g.,* Kopelman, Nature 404: 635-43 (2000)).

Obesity reduces life-span and carries a serious risk of the co-morbidities listed above, as well disorders such as infections, varicose veins, acanthosis nigricans, eczema, exercise intolerance, insulin resistance, hypertension hypercholesterolemia, cholelithiasis, orthopedic injury, and thromboembolic disease (Rissanen et al., Br. Med. J. 301: 835-7 (1990)). Obesity is also a risk factor for the group of conditions called insulin resistance syndrome, or "Syndrome X" and metabolic syndrome. The worldwide medical cost of obesity and associated disorders is enormous.

The pathogenesis of obesity is believed to be multifactorial. A problem is that, in obese subjects, nutrient availability and energy expenditure do not come into balance until there is excess adipose tissue. The central nervous system (CNS) controls energy balance and coordinates a variety of behavioral, autonomic and endocrine activities appropriate to the metabolic status of the animal. The mechanisms or systems that control these activities are broadly distributed across the forebrain (e.g., hypothalamus), hindbrain (e.g., brainstem), and spinal cord. Ultimately, metabolic (i.e., fuel availability) and cognitive (i.e., learned preferences) information from these systems is integrated and the decision to engage in appetitive (food seeking) and consummatory (ingestion) behaviors is either turned on (meal procurement and initiation) or turned off (meal germination). The hypothalamus is thought to be principally responsible for integrating these signals and then issuing commands to the brainstem. Brainstem nuclei that control the elements of the consummatory motor control system (e.g., muscles responsible for chewing and swallowing). As such, these CNS nuclei have literally been referred to as constituting the "final common pathway" for ingestive behavior.

Neuroanatomical and pharmacological evidence support that signals of energy and nutritional homeostatis integrate in forebrain nuclei and that the consummatory motor control system resides in brainstem nuclei, probably in regions surrounding the trigeminal motor nucleus. There are extensive reciprocal connection between the hypothalamus and brainstem. A variety of CNS-directed anti-obesity therapeutics (e.g., small molecules and peptides) focus predominantly upon forebrain substrates residing in the hypothalamus and/or upon hindbrain substrates residing in the brainstem.

Obesity remains a poorly treatable, chronic, essentially intractable metabolic disorder. Accordingly, a need exists for new therapies useful in weight reduction and/or weight maintenance in a subject. Such therapies would lead to a profound beneficial effect on the subject's health.

### SUMMARY

The present invention provides the subject matter of the claims.

### BRIEF DESCRIPTION OF THE DRAWINGS

Fig. 1 is a graph depicting an effect of administration of leptin and amylin on food intake.

Fig. 2 is a graph depicting an effect of administration of leptin and amylin on body weight.

Fig. 3 is a graph depicting an effect of administration of leptin and amylin on body composition.

Fig. 4 is a graph depicting an effect of administration of leptin and amylin on body composition.

Fig. 5 is a graph depicting an effect of administration of leptin and amylin on energy expenditure.

Fig. 6A is a graph depicting an effect on body weight of administration of leptin (500 µg/kg/day) and amylin (100 µg/kg/day), either alone or in combination over two weeks. Fig. 6B is a graph depicting an effect on body fat of a two-week administration of leptin (500 µg/kg/day) and amylin (100 µg/kg/day), either alone or in combination. Fig. 6C is a graph depicting an effect on body protein of a two-week administration of leptin (500 µg/kg/day) and amylin (100 µg/kg/day), either alone or incombination.

Fig. 7 is a graph depicting an effect on body weight of administration of leptin alone (500 µg/kg/day), pair-fed leptin alone (500 µg/kg/day), and leptin (500 µg/kg/day and amylin (100 µg/kg/day) in combination over two weeks.

Fig. 8 shows graphs depicting serum leptin concentrations in normal HSD and in DIO prone animals that either received vehicle, were pair-fed to the amylin-treated group, or received amylin (100 µg/kg/day) for two weeks.

Fig. 9A is a graph depicting an effect on body weight of administration of vehicle or leptin (500 µg/kg/day) in normal animals. Fig. 9B is a graph depicting aneffect on body weight of administration of vehicle or leptine (500 µg/kg/day) in DIO prone animals.

Fig. 10A is a graph depicting an effect on body weight of administration of sibutramine (3 mg/kg/day) and amylin (100 µg/kg/day), either alone or in combination over two weeks. Fig. 6B is a graph depicting an effect on body fat of a two-week administration of sibutramine (3 mg/kg/day) and amylin (100 µg/kg/day), either alone or in combination. Fig. 6C is a graph depicting an effect on body protein of a two-week administration of sibutramine (3 mg/kg/day) and amylin (100 µg/kg/day), either alone or in combination.

Fig. 11A is a graph depicting an effect on body weight of administration of phentermine (10 mg/kg/day) and amylin (100 µg/kg/day), either alone or in combination over two weeks. Fig. 6B is a graph depicting an effect on body fat of a two-week administration of phentermine (10 mg/kg/day) and amylin (100 µg/kg/day), either alone or in combination. Fig. 6C is a graph depicting an effect on body protein of a two-week administration of phentermine (10 mg/kg/day) and amylin (100 µg/kg/day), either alone or in combination.

Fig. 12A is a graph depicting an effect on body weight of administration of rimonabant (3 mg/kg/day) and amylin (100 µg/kg/day), either alone or in combination over two weeks. Fig. 6B is a graph depicting an effect on body fat of a two-week administration of rimonabant (3 mg/kg/day) and amylin (100 µg/kg/day), either alone or in combination. Fig. 6C is a graph depicting an effect on body protein of a two-week administration of rimonabant (3 mg/kg/day) and amylin (100 µg/kg/day), either alone or in combination.

Figure 13 is a graph depicting the effect of administration of a range of doses of a CB-1 antagonist, either alone or in combination with amylin (100 µg/kg/day), on body weight. The time course of the combinations in the circled area are depicted in Figs. 14A and B.

Fig. 14A is a graph depicting the effect of administration of a CB-1 antagonist (1 1 mg/kg/day) and amylin (100 µg/kg/day), either alone or in combination, on body weight Fig. 14B is a graph depicting the effect of administration of a CB-1 antagonist (3 mg/kg/day) and amylin (100 µg/kg/day), either alone or in combination, on body weight.

Figure 15A is a graph depicting an effect on body weight of administration of an exendin-4 analog (10 µg/kglclay) and amylin (100 µg/kg/day), either alone or in combination over two weeks. Fig. 6B is a graph depicting an effect on body fat of a two-week administration of an exendin-4 analog (10 µg/kg/day) and amylin (100 µg/kg/day), either alone or in combination. Fig. 6C is a graph depicting an effect on body protein of a two-week administration of exendin-4 (10 µg/kg/day) and amylin (100 µg/kg/day), either alone or in combination.

Figure 16A is a graph depicting an effect on body weight of administration of PYY(3-36) (1 mg/kg/day) and amylin (100 µg/kg/day), either alone or in combination over two weeks. Fig. 6B is a graph depicting an effect on body fat of a two-week administration of PYY(3-36)(1 mg/kg/day) and amylin (100 µg/kg/day), either alone or in combination. Fig. 6C is a graph depicting an effect on body protein of a two-week administration of PYY(3-36) (1 mg/kg/day) and amylin (100 µg/kg/day), either alone or in combination.

### DETAILED DESCRIPTION

We have discovered that administration of an anti-obesity agent that acts upon structures in the forebrain involved in food intake and/or body weight modulation in combination with an anti-obesity agent that acts upon structures in the hindbrain involved in food intake and/or body weight modulation is suprisingly effective in reducing nutrient availability and in treating obesity and obesity related conditions, disorders, and diseases. It has been discovered that when administration of such anti-obesity agents is combined in this manner, the anti-obesity agents are more effective in reducing nutrient availability in the recipient than use of one of the agents alone. As shown herein, for example, a combination of anti-obesity agents can act synergistically to reduce nutrient availability, e.g., to reduce weight, reduce fat, reduce food intake, or any combination of these three.

Particular areas of the forebrain (telencephalonic- and diencephalonic-derived constituents of the brain) and hindbrain or brainstem (including the midbrain, pons and medulla) have been identified as being involved in controlling energy balance. Forebrain structures or nuclei residing in the hypothalamus involved in food intake and/or body weight modulation include, for example, the arcuate nucleus (ARC), the paraventricular nucleus (PVN), the dorsomedial hypothalamus (DMH), the ventromedial nucleus (VMH), and the lateral hypothalamus nucleus (LHA). Hindbrain structures or nuclei residing in the brainstem involved in food intake and/or body weight modulation include, for example, the nucleus of the solitary tract (NST), the area postrema (AP), and the lateral parabrachial nucleus (1PBN). Brainstem nuclei that control the elements of the consummatory motor control system are likely controlled by primary or second order projections from brainstem regions like the NST, AP, and IPBN. It is noteworthy that the AP, NST and 1PBN have all been shown to (collectively and independently) possess their own integrative abilities.

A variety of CNS-directed anti-obesity agents act upon these forebrain structures residing in the hypothalamus involved in food intake and/or body weight modulation. In addition, CNS-directed anti-obesity agents act upon hindbrain structures residing in the brainstem involved in food intake and/or body weight modulation. Examples of such anti-obesity agents are described herein. See Table 1 for examples. Such agents include, for example, neuropeptide Y1 (NPY1) receptor antagonists, NPY5 receptor antagonists, leptin and leptin agonists, ciliary neurotrophic factor (CNTF) and CNTF agonists, melanin-concentrating hormone (MHC) and MCH antagonists, melacortins (MC) and MC agonists, cannabinoid receptor (CB-1) antagonists, serotonin (5-HT) and 5-HT agonists, peptide YY (PYY) and PYY agonists, exendin and exendin agonists, GLP-1 and GLP-1 agonist, DPP-IV inhibitors, ghrelin and ghrelin antagonists, cholecystokinin (CCK) and CCK agonists, and amylin and amylin agonists.

The effects of forebrain targeted agent in combination with hindbrain targeted agent has been reported for a variety of indications. For example, the effect of a non-peptide MCH antagonist (forebrain target) in combination with a CCK agonist (hindbrain target) has reportedly been studied with regard to treatment of obesity and/or anorexia (PCT Publication No. WO 03/047568). Additional examples of combinations of forebrain and hindbrain targeted agents include the following: MCH-1 R antagonist in combination with CCK agonist for use in the treatment or prevention of obesity (PCT Publication No. WO 03/045920); MCH antagonist in combination with CCK agonist for treatment of obesity and appetite regulation (US Application Publication No. US 2004/0132752); exendin or exendin agonist in combination with CCK or amylin agonist for treatment of obesity and appetite reduction (PCT Publication No. 00/66629); exendin or exendin agonist in combination with a CCK, amylin or amylin agonist for treating conditions or disorders which can be alleviated by reducing food intake (PCT Publication No. 98/30231); GLP-1 in combination with CCK-33 for reduction in food intake (Gutzwiller et al., 2004, Amer. J. Physiology Regulatory, Integrative and Comparative Physiology 287:R562-R567); GLP-1 agonist in combination with CCK agonist for treatment of obesity (US Application Publication No. US 2003/0220255); GLP-1 derivative in combination with CCK agonist for treatment of obesity (US 6,458,924); PYY or agonist thereof in combination with amylin and amylin agonist for reduction in food intake or appetite reduction (PCT Publication No. WO 03/057235); NPY5 receptor antagonist in combination with CCK agonist for treatment of obesity (US 6,313,298; PCT Publication No. WO 2004/009015); leptin in combination with CCK for treatment of obesity (Matson et al., 1997, Peptides 18:1275-1278; Matson et al., 2000, Amer. J. Physiology Regulatory, Integrative and Comparative Physiology 278:R882-R890); leptin or exendin(1-39) in combination with CCK-8 for inhibition of food intake (US Application Publication No. US 2004/0116657); 5-HT2c receptor ligand, agonist or antagonist in combination with CCK agonist for treatment of obesity (PCT Publication No. WO 03/000663; PCT Publication No. WO 2004/056324).

**Table 1. Individual anti-obesity targets and location**

| Signaling System | CNS Region | Food Intake Role | Anti-obesity agents |
|---|---|---|---|
| Neuropeptide Y (NPY) | Forebrain (ARC/PVN) | Increases intake | NPY1 and NPY5 receptor antagonists |
| Leptin | Forebrain (ARC) | Decreases intake | Leptin, or agonists |
| Ciliary neurotrophic factor (CNTF) | Forebrain (ARC) | Decreases intake | CNTF (Axokine®) |
| Melanin-concentrating hormone (MCH) | Forebrain (ARC/PVN) | Increases intake | MCH antagonists |
| Melanocortins (MC) | Forebrain (PVN/ARC) | Agonists decrease intake | MC4 agonists |
| Cannabinoids (CB) | Forebrain (widespread) | Increase intake | Cannabinoid receptor antagonists |
| Serotonin (5-HT) | Forebrain (VMH) | Decrease intake | 5-HT2C agonists |
| Peptide YY (PYY) | Forebrain (ARC) | Decrease intake | PYY(3-36) agonists |
| Glucagon-like peptide-1 (GLP-1) | Forebrain (PVN) | Decrease intake | Exenatide and other GLP-1 ligands, DPP-IV inhibitors |
| Ghrelin | Forebrain (ARC) | Increase intake | Ghrelin antagonists |
| Cholecystokinin (CCK) | Hindbrain (AP) | Decrease intake | CCK agonists |
| Amylin | Hindbrain (AP) | Decrease intake | Amylin agonists, Pramlintide, amylin analogs |

The first compound predominantly targets the energy balance centers of the hypothalamus, such as the ARC, PVN, VM, and LH. The second compound predominantly targets the energy balance centers of the hindbrain such as the the AP and the 1PBN.

These compounds are anti-obesity agents. The invention includes use of one or more predominantly forebrain acting anti-obesity agents. The invention also includes use of one or more predominantly hindbrain acting anti-obesity agents. The anti-obesity agents include a leptin or a leptin agonist or derivative, and an amylin or an amylin agonist having at least 80 % amino acid sequence identity to SEQ ID NO :1 having amylin activity.

As exemplified herein, agonists that are anti-obesity agents in the invention include, for example, molecules such peptides, polypeptides and small molecules agents.

The details of one or more embodiments of the invention are set forth in the accompanying drawings and the description below. Other features, objects, and advantages of the invention will be apparent from the description and drawings, and from the claims.

### Definitions

An "anti-obesity agent" is a compound that is able to reduce nutrient availability to the body upon administration. A "weight-inducing agent" is a compound that would increase nutrient availability to the body. In one aspect, a weight-inducing agent is an antagonist of an anti-obesity agent.

As used herein, an anti-obesity agent that "acts on a forebrain structure involved in food intake and/or body weight modulation" stimulates or suppresses activity of a particular region, e.g., particular nuclei and/or neuronal circuits, in the forebrain. This forebrain stimulation or suppression leads to a reduction in nutrient availability to the body. An anti-obesity agent that "acts on a hindbrain structure involved in food intake and/or body weight modulation" stimulates or suppresses activity of a particular region, e.g., particular nuclei and/or neuronal circuits, in the hindbrain. This hindbrain stimulation or suppression results in a reduction in nutrient availability to the body.

"Reduced nutrient availability" is meant to include any means by which the body reduces the nutrients available to the body to store as fat. In other words, reducing nutrient availability may be by means that include, but are not limited to, reducing appetite, increasing satiety, affecting food choice/taste aversion, increasing metabolism, and/or decreasing or inhibiting food absorption. Exemplary mechanisms that may be affected include delayed gastric emptying or decreased absorption of food in the intestines.

"Increased nutrient availability" is meant to include any means by which the body increases the nutrients available to the body to store as fat In other words, increasing nutrient availability may be by means that include, but are not limited to, increasing appetite, decreasing satiety, affecting food choice, decreasing taste aversion, decreasing metabolism, and/or increasing food absorption. Exemplary mechanisms that may be affected include decreasing gastric hypomotility or increasing absorption of food in the intestines.

While "obesity" is generally defined as a body mass index (BMI) over 30, for purposes of this disclosure, any subject, including those with a body mass index of less than 30, who needs or wishes to reduce body weight or prevent body weight gain is included in the scope of "obese" Thus, subjects with a BMI of less than 30 and 25 and above (considered overweight) or below 25 are also included in the subjects of the invention. Morbid obesity refers to a BMI of 40 or greater.

With regard to reduction of nutrient availability, as used herein, a "subject in need thereof includes subjects who are overweight or obese or morbidly obese, or desirous of losing weight. In addition, subjects who are insulin resistant, glucose intolerant, or have any form of diabetes mellitus (e.g., type 1, 2 or gestational diabetes) can benefit to reduce nutrient availability.

With regard to increase of nutrient availability, as used herein, a "subject in need thereof" includes subjects who are underweight or desirous of gaining weight

A "subject" is meant to be human who has a serum leptin concentration prior to administration of the anti-obesity agents of greater than 10 ng/ml.

By "metabolic rate" is meant the amount of energy liberated/expended per unit of time. Metabolism per unit time can be estimated by food consumption, energy released as heat, or oxygen used in metabolic processes. It is generally desirable to have a higher metabolic rate when one wants to loose weight. For example, a person with a high metabolic rate may be able to expend more energy (e.g., the body bums more calories) to perform an activity than a person with a low metabolic rate for that activity.

As used herein, "lean mass" or "lean body mass" refers to muscle and bone. Lean body mass does not necessarily indicate fat free mass. Lean body mass contains a small percentage of fat (roughly 3%) within the central nervous system (brain and spinal cord), marrow of bones, and internal organs. Lean body mass is measured in terms of density. Methods of measuring fat mass and lean mass include, but are not limited to, underwater weighing, air displacement plethysmograph, x-ray, DEXA scans, MRIs and CT scans. In one embodiment, fat mass and lean mass is measured using underwater weighing.

By "fat distribution" is meant the location of fat deposits in the body. Such locations of fat deposition include, for example, subcutaneous, visceral and ectopic fat depots.

By "subcutaneous fat" is meant the deposit of lipids just below the skin's surface. The amount of subcutaneous fat in a subject can be measured using any method available for the measurement of subcutaneous fat. Methods of measuring subcutaneous fat are known in the art, for example, those described in U.S. Patent No. 6,530,886.

By "visceral fat" is meant the deposit of fat as intra-abdominal adipose tissue. Visceral fat surrounds vital organs and can be metabolized by the liver to produce blood cholesterol. Visceral fat has been associated with increased risks of conditions such as polycystic ovary syndrome, metabolic syndrome and cardiovascular diseases.

By "ectopic fat storage" is meant lipid deposits within and around tissues and organs that constitute the lean body mass (e.g., skeletal muscle, heart, liver, pancreas, kidneys, blood vessels). Generally, ectopic fat storage is an accumulation of lipids outside classical adipose tissue depots in the body.

As used herein, and as well-understood in the art, "treatment" is an approach for obtaining beneficial or desired results, including clinical results. "Treating" or "palliating" a disease, disorder, or condition means that the extent and/or undesirable clinical manifestations of a condition, disorder, or a disease state are lessened and/or time course of the progression is slowed or lengthened, as compared to not treating the disorder. For example, in treating obesity, a decrease in body weight, e.g., at least a 5% decrease in body weight, is an example of a desirable treatment result. For purposes of this invention, beneficial or desired clinical results include, but are not limited to, alleviation or amelioration of one or more symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Further, treating does not necessarily occur by administration of one dose, but often occurs upon administration of a series of doses. Thus, a therapeutically effective amount, an amount sufficient to palliate, or an amount sufficient to treat a disease, disorder, or condition may be administered in one or more administrations.

As used herein, the term "therapeutically effective amount" means the amount of the active compounds in the composition that will elicit the biological or medical response in a tissue, system, subject, or human that is being sought by the researcher, veterinarian, medical doctor or other clinician, which includes alleviation of the symptoms of the disorder being treated provided that the anti-obesity agents are administered in amounts effective to reduce the body weight of the subject by at least 10 %. The novel uses of this invention are for disorders known to those skilled in the art.

As used herein, the term "prophylactically effective amount" means the amount of the active compounds in the composition that will elicit the biological or medical response in a tissue, system, subject, or human that is being sought by the researcher, veterinarian, medical doctor or other clinician, to prevent the onset of obesity or an obesity-related disorder, condition or disease in subjects as risk for obesity or the obesity-related disorder, condition or disease.

As used herein, the singular form "a", "an", and "the" includes plural references unless otherwise indicated or clear from context. For example, as will be apparent from context, "an" amylin agonist can include one or more amylin agonists.

As used herein, an "analog" refers to a peptide whose sequence was derived from that of a base reference peptide, amylin and includes insertions, substitutions, extensions, and/or deletions of the reference amino acid sequence, having at least 80%, 90%, or 95% amino acid sequence identity with the base peptide. Such analogs may comprise conservative or non-conservative amino acid substitutions (including non-natural amino acids and L and D forms). Analogs are compounds having agonist activity. Analogs, as herein defined, also include derivatives.

A "derivative" is defined as a reference peptide or analogs, described above, having a chemical modification of one or more of its amino acid side groups, a-carbon atoms, terminal amino group, or terminal carboxylic acid group. A chemical modification includes, but is not limited to, adding chemical moieties, creating new bonds, and removing chemical moieties. Modifications at amino acid side groups include, without limitation, acylation of lysine ε-amino groups, N-alkylation of arginine, histidine, or lysine, alkylation of glutamic or aspartic carboxylic acid groups, and deamidation of glutamine or asparagine. Modifications of the terminal amino include, without limitation, the desamino, N-lower alkyl, N-di-lower alkyl, and N-acyl modifications. Modifications of the terminal amino include, without limitation, the desamino, N-lower alkyl, N-di-lower alkyl, and N-acyl modifications, such as alkyl acyls, branched alkylacyls, alkylaryl-acyls. Modifications of the terminal carboxy group include, without limitation, the amide, lower alkyl amide, dialkyl amide, arylamide, alkylarylamide and lower alkyl ester modifications. Lower alkyl is C1-C4 alkyl. Furthermore, one or more side groups, or terminal groups, may be protected by protective groups known to the ordinarily-skilled synthetic chemist. The α-carbon of an amino acid may be mono- or dimethylated.

In general, with respect to an amino acid sequence, the term "modification" includes substitutions, insertions, elongations, deletions, and derivatizations alone or in combination. The polypeptides of the invention may include one or more modifications of a "non-essential" amino acid residue. In the context of the invention, a "non-essential" amino acid residue is a residue that can be altered, e.g., deleted or substituted, in the novel amino acid sequence without abolishing or substantially reducing the activity (*e*.*g*., the agonist activity) of the polypeptide (e.g., the analog polypeptide). The polypeptides of the invention may include deletions of 1,2,3,4,5,6,7,8,9,10 or more non-essential amino acid residues. The polypeptides of the invention may include additions of at least of 1,2,3, 4, 5, 6, 7, 8,9,10 or more amino acids without abolishing or substantially reducing the activity of the polypeptide.

Substitutions include conservative amino acid substitutions. A "conservative amino acid substitution" is one in which the amino acid residue is replaced with an amino acid residue having a similar side chain, or physicochemical characteristics (e.g., electrostatic, hydrogen, bonding, isosteric, hydrophobic features). The ammo acids may be naturally occurring or nonnatural (unnatural). Families of amino acid residues having similar side chains are known in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, methionine, cysteine), nonpolar side chains (e.g., alanine, valine, leucine, isoleucine, proline, phenylalanine, tryptophan), β-branched side chains (e.g., threonine, valine, isoleucine) and aromatic side chains (e.g., tyrosine, phenylalanine, tryptophan, histidine). Substitutions may also include non-conservative changes.

By "amino acid" or "amino acid residue" is meant natural amino acids, unnatural amino acids, and modified amino acid. Unless stated to the contrary, any reference to an amino acid, generally or specifically by name, includes reference to both the D and the L stereoisomers if their structure allow such stereoisomeric forms. Natural amino acids include alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), Lysine (Lys), methionine (Met), phenylalanine (Phe), proline (Pro), serine (Ser), threonine (Thr), tryptophan (Trp), tyrosine (Tyr) and valine (Val). Unnatural amino acids include, but are not limited to homolysine, homoarginine, homoserine, azetidinecarboxylic acid, 2-aminoadipic acid, 3-aminoadipic acid, beta-alanine, aminopropionic acid, 2-aminobutyric acid, 4-aminobutyric acid, 6-aminocaproic acid, 2-aminoheptanoic acid, 2-aminoisobutyric acid, 3-aminoisbutyric acid, 2-aminopimelic acid, tertiary-butylglycine, 2,4-diaminoisobutyric acid, desmosine, 2,2'-diaminopimelic acid, 2,3-diaminopropionic acid, N-ethylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, pentylglycine, pipecolic acid and thioproline. Additional unnatural amino acids include modified amino acid residues which are chemically blocked, reversibly or irreversibly, or chemically modified on their N-terminal amino group or their side chain groups, as for example, N-methylated D and L amino acids or residues wherein the side chain functional groups are chemically modified to another functional group. For example, modified amino acids include methionine sulfoxide; methionine sulfone; aspartic acid- (beta-methyl ester), a modified amino acid of aspartic acid; N-ethylglycine, a modified amino acid of glycine; or alanine, carboxamide, a modified amino acid of alanine. Additional residues that can be incorporated are described in Sandberg et al., J. Med. Chem. 41:2491-91, 1998.

It should be noted that throughout the application that alternatives are written in Markush groups, for example, each amino acid position that contains more than one possible amino acid. It is specifically contemplated that each member of the Markush group should be considered separately, thereby comprising another embodiment of the invention, and the Markush group is not to be read as a single unit.

### Uses of the Invention

The invention provides a combination useful for treating obesity and obesity-related diseases, disorders, and/or conditions that would benefit from a reduction in nutrient availability in the subjects defined in the claims. Given the increase in effectiveness when used in combinations, the invention may allow for administration of lower dosages of one or more of the anti-obesity agents used in combination as compared to the use of the agent alone, such as in monotherapy.

The invention is based on administration of a combination of anti-obesity agents. Administration of the agents "in combination" should be understood to mean providing each of the agents to a subject in need of treatment. Administration of the agents could occur as a single pharmaceutical dosage formulation containing all of the intended anti-obesity agents or in separately with each intended agent in its own dosage formulation.

Where separate dosage formulations are used, the individual anti-obesity agents can be administered at essentially the same time, *i.e.,* concurrently, or at separately staggered times, *i.e.*, sequentially prior to or subsequent to the administration of the other anti-obesity agent. In some embodiments, administration in combination involves administration of separate dosage formulations during overlapping intervals. For example, anti-obesity agent 1 is administered from day 1 through day 30 and anti-obesity agent 2 is administered from day 20 through day 50. In other embodiments, administration in combination involves administration of separate dosage formulations in sequential, nonoverlapping intervals. For example, anti-obesity agent 1 is administered from day 1 through day 30 and anti-obesity agent 2 is administered from day 35 through day 50. The instant invention is therefore to be understood to include all such regimes of simultaneous, alternating, or completely separate treatments over the total treatment course, and the terms "administration," "administering," "administration in combination" and "administering in combination" are to be interpreted accordingly.

In some cases, the invention increases or enhances the effectiveness an anti-obesity agent that has limited effectiveness, if any, when used alone (monotherapy). In such cases, the invention increases or enhances the effectiveness an anti-obesity agent by, for example, preventing or delaying loss of effectiveness by continued use or increasing potency. Uses according to the invention may allow for administration of lower dosages of one or more of the anti-obesity agents used in combination as compared to the use of either agent alone.

The invention provides a synergistic anti-obesity effect among the administered agents. Accordingly, in one embodiment, administration of a combination of anti-obesity agents results in a reduction in body weight, which is greater than the combination of the results of administration of the anti-obesity agent alone (monotherapy) and is at least 10 %.

In another aspect of the invention, a subject's resistance to an anti-obesity agent is reduced or eliminated so that when the agent is administered, it will be able to elicit an anti-obesity response (e.g., reduce nutrient availability, reduce weight, reduce fat mass). For example, and without wishing to be bound by this or any other theory, it is theorized that leptin resistance may be due to the high levels of leptin found in obese subjects (i.e., the body has become desensitized to leptin). Therefore, one method of the invention comprises administering an anti-obesity agent other than leptin (e.g., amylin or an amylin agonist) to reduce weight of the subject so as to reduce or remove the leptin resistance. Once this has been achieved, leptin is then to be administered, either alone or in combination with an anti-obesity agent (e.g., amylin or an amylin agonist), for a further anti-obesity effect. Other means of reducing weight to ameliorate leptin resistance are contemplated, such as diet, exercise, other diet drugs, and surgical devices.

In one embodiment, the invention allows for the delivery of the first anti-obesity agent that acts upon hindbrain structures involved in food intake and/or body weight modulation to prime the body before administration of the second anti-obesity agent that acts upon forebrain structures involved in food intake and/or body weight modulation. In certain embodiments, the administration of the first agent is for a number of days, weeks or even months before the administration of the second agent. At this point, the second agent may be administered alone or in combination with the first agent. The first anti-obesity agent is amylin or an amylin agonist and the second agent is a leptin or a leptin agonist. The serum leptin concentration of a subject prior to administration of the anti-obesity agents is greater than 10 ng/ml, in other embodiments, it is greater than 20 ng/ml.

Herein are/is described use of a combination of anti-obesity agents for manufacture of a medicament for reducing the risk of developing metabolic disorders are provided, where the medicament is to be administered to the subject in effective amounts to reduce the weight of a subject by at least 10 %.

Herein are/is described, medicaments are used to increase the metabolic rate in a subject, decrease a reduction in the metabolic rate in a subject, or preserve the metabolic rate in a subject provided that the anti-obesity agents are administered in amounts effective to reduce the body weight of the subject by at least 10%. In one embodiment, the metabolic rate may involve the preferential use of the body's fat as an energy source over lean body tissue. In one aspect, lean body mass is not decreased following administration of the combination of anti-obesity agents. In another aspect, a reduction in the lean body mass is lessened or prevented following administration of the combination of anti-obesity agents. In still another aspect, lean body mass is increased following administration of the combination of anti-obesity agents. Such preference for fat as the energy source may be determined by comparing the amount of fatty tissue to lean body tissue, ascertained by measuring total body weight and fat content at the beginning and end of the treatment period. An increase in metabolic rate is a higher level of the use of calories or another energy source by a subject over a period of time compared with the level of use of calories or other energy source by the subject over another period of time under substantially similar or identical conditions without administration of the combination of anti-obesity agents. In one embodiment, the metabolic rate is increased at least about 5% in a subject, in other embodiments, the metabolic rate is increased at least about 10%, 15%, 20% 25%, 30%, or 35% in a subject compared with the level of use of calories or other energy source by the subject over another period of time under substantially similar or identical conditions without administration of the combination of anti-obesity agents. The increase in metabolic rate can be measured using a respiratory calorimeter, for example. An effective amount of the anti-obesity agents of as used in this embodiment is an amount of each agent effective to increase the metabolic rate in a subject when administered in combination compared to a subject not receiving the agents or only one of the agents.

Herein are/is described, the medicament can also reduce a decrease in metabolic rate in a subject provided that the anti-obesity agents are administered in amounts effecctive to reduce the body weight of the subject by at least 10 %. Such a decrease in metabolic rate can be the result of any condition or nutritional or physical regimen that leads to a reduction in metabolic rate, for example, due to a reduced calorie diet, a restricted diet, or weight loss. A restricted diet includes allowances or prohibitions, or both on the types of food or the amounts of food or both permitted in a diet, not necessarily based on calories. For example, as in individual diets, the body compensates with a reduced metabolic rate based on the lower caloric intake. In essence, the body down-regulates the requirement for food, thereby subsisting on less food. As dieting continues, the threshold for caloric intake is reduced. When dieting has ended, the individual typically gains weight while eating a normal diet because of the lowered caloric intake threshold and lower-basal metabolic rate (NIH Technology Assessment Conference Panel (1992) Ann. Intern. Med. 116:942-949; Wadden (1993) Ann. Intern. Med. 119:688-693). In one aspect, the loss of metabolic rate in a subject is reduced, where the loss of metabolic rate is the result of a reduced calorie diet or weight loss. The subject's reduction in metabolic rate is decreased by at least about 10%, 15%, 20% 25%, 30%, 35%, 40%, 50%, 60%, 70%, 80%, 9%, or 95% in a subject. It may be desirable to formulate the medicament for administration of the combination of anti-obesity agents at the time the condition or nutritional or physical regimen is initiated which leads to a loss or reduction in metabolic rate. However, it is also contemplated that administration of the agents is commenced before the condition or nutritional or physical regimen is initiated. In one instance, metabolic rate is measured using a respiratory calorimeter. An effective amount of the anti-obesity agents of as used in this embodiment is an amount of each agent effective to decrease the reduction of the metabolic rate in a subject when administered in combination.

Herein are/is described, uses for reducing metabolic plateaus are provided, comprising administration of effective amounts of anti-obesity agents in combination to a human subject provided that the anti-obesity agents are administered in amounts effective to reduce the body weight of the subject by at least 10 %. Herein are/is described, the subject is losing weight, or has lost weight, for example, due to a reduced calorie diet, increased exercise or a combination thereof. By "metabolic plateau" is meant time intervals of steady metabolic rate while the body adjusts to changes in caloric or energy input Changes in caloric input or expenditure can be the result of, for example, reduced calorie diets or increased physical activity. Such plateaus can be observed, for example, during a weight loss regimen when weight loss slows or stops. Herein are/is described, use reduce the duration of a metabolic plateau in a subject compared with the duration of metabolic plateaus in an otherwise identical subject over the same period of time under substantially similar or identical conditions without administration of the combination of anti-obesity agents. Herein are/is described, uses reduce the frequency of metabolic plateaus compared with the frequency of metabolic plateaus in an otherwise identical subject over the same period of time under substantially similar or identical conditions without administration of the combination of anti-obesity agents. Herein are/is described, a use delays the onset of a metabolic plateau compared with the onset of a metabolic plateau in an otherwise identical subject over the same period of time under substantially similar or identical conditions without administration of the combination of anti-obesity agents. Herein are/is described, metabolic plateaus are identified by charting periods of reduced or no weight loss. Herein are/is described, at least one metabolic plateau is reduced. In other embodiments, at least two, three, four, five, six, seven, eight, nine, or ten metabolic plateaus are reduced. In another aspect, metabolic plateaus are delayed one day as compared to a subject not administered the combination of anti-obesity agents under identical or similar conditions. In other aspects, metabolic plateaus are delayed 2 days, 3 days, 4 days, 5 days, 6 days, 1 week, 10 days, 2 weeks or 3 weeks in a subject.

Herein are/is described, a use is provided to preserve the metabolic rate in a human subject provided that the anti-obesity agents are administered in amounts effective to reduce the body weight of the subject by at least 10%. The subject may be at risk of losing metabolic rate, for example, due to the initiation of a reduced calorie diet, restricted diet, or anticipated weight loss. A preservation of metabolic rate is a maintenance of the level of the use of calories or another energy source by a subject over a period of time compared with the level of use of calories or other energy source by an otherwise identical subject over the same period of time under substantially similar or identical conditions without administration of the combination of anti-obesity agents. In one aspect, the metabolic rate is maintained within 15% of the subject's metabolic rate prior to the initiation of the event that results in the decrease in metabolic rate. In other aspects, the metabolic rate is maintained within 10%, within 7%, within 5%, within 3% or less of the subject's metabolic rate. In one aspect, the combination of anti-obesity agents is administered at the initiation of a reduced calorie diet, restricted diet, or exercise regimen.

Metabolic rates can be assessed using any method available for determining such rates, for example by using a respiratory calorimeter. Such methods and devices for assaying metabolic rates are known in the art and are described, for example, in U.S. Patent Nos. 4,572,208, 4,856,531, 6,468,222, 6,616,615, 6,013,009, and 6,475,158. Alternatively, the metabolic rate of an animal can be assessed by measuring the amount of lean tissue versus fatty tissue catabolized by the animal following the diet period. Thus, total body weight and fat content can be measured at the end of the dietary period. In rats, a frequently used method to determine total body fat is to surgically remove and weigh the retroperitoneal fat pad, a body of fat located in the retroperitoneum, the area between the posterior abdominal wall and the posterior parietal peritoneum. The pad weight is considered to be directly related to percent body fat of the animal. Since the relationship between body weight and body fat in rats is linear, obese animals have a correspondingly higher percent of body fat and retroperitoneal fat pad weight

In another aspect of the present invention, uses for reducing fat mass by increasing the metabolic rate in a subject are provided, where a combination of anti-obesity agents is to be administered in amounts effective to reduce fat mass by increasing the subject's metabolic rate provided that the anti-obesity agents are administered in amounts effective to reduce the body weight of the subject by at least 10%. Fat mass can be expressed as a percentage of the total body mass. In some aspects, the fat mass is reduced by at least 1%, at least 5%, at least 10%, at least 15%, at least 20%, or at least 25% oyer the course of treatment. In one aspect, the subject's lean mass is not decreased over the course of the treatment. In another aspect, the subject's lean mass is maintained or increased over the course of the treatment. In another aspect, the subject is on a reduced calorie diet or restricted diet. By "reduced calorie diet" is meant that the subject is ingesting fewer calories per day than compared to the same subject's normal diet. In one instance, the subject is consuming at least 50 fewer calories per day. In other instances, the subject is consuming at least 100, 150, 200, 250, 300, 400, 500, 600, 700, 800, 900, or 1000 fewer calories per day.

In one embodiment of the present invention, a use for altering the fat distribution in a subject is provided where a combination of anti-obesity agents is to be administered in amounts effective to alter fat distribution in the subject provided that the anti-obesity agents are administered in amounts effective to reduce the body weight of the subject by at least 10%. In one aspect, the alteration results from an increased metabolism of visceral or ectopic fat, or both in the subject. In some embodiments, the metabolism of visceral or ectopic fat or both is at a rate of at least about 5%, 10%, 15%, 20%, 25%, 30%, 40%, or 50% greater than for subcutaneous fat In one aspect, the uses result in a favorable fat distribution. In one embodiment, favorable fat distribution is an increased ratio of subcutaneous fat to visceral fat, ectopic fat, or both. In one aspect, this involves an increase in lean body mass, for example, as a result of an increase in muscle cell mass.

In another embodiment, uses for reducing the amount of subcutaneous fat in a subject are provided, wherein to a subject in need thereof, a combination of anti-obesity agents is to be administered in amounts effective to reduce the amount of subcutaneous fat in the subject provided that the anti-obesity agents are administered in amounts effective to reduce the body weight of the subject by at least 10%. In one instance, the amount of subcutaneous fat is reduced in a subject by at least about 5%. In other instances, the amount of subcutaneous fat is reduced by at least about 10%, 15%, 20%, 25%, 30% 40%, or 50% compared to the subject prior to administration of the anti-obesity agents.

The used described herein can be used to reduce the amount of visceral fat in a subject. In one instance, the visceral fat is reduced in a subject by at least about 5%. In other instances, the visceral fat is reduced in the subject by at least about 10%, 15%, 20%, 25%, 30% 40%, or 50% compared to the subject prior to administration of the combination of anti-obesity agents provided that the anti-obesity agents are administered in amounts effective to reduce the body weight of the subject by at least 10%.. Visceral fat can be measured through any means available to determine the amount of visceral fat in a subject. Such methods include, for example, abdominal tomography by means of CT scanning and MRI. Other methods for determining visceral fat are described, for example, in U.S. Patent Nos. 6,864,415, 6,850,797, and 6,487,445.

In one embodiment, a use for preventing the accumulation of ectopic fat or reducing the amount of ectopic fat in a subject is provided, wherein to a subject in need thereof, a combination of anti-obesity agents is to be administered in amounts effective to prevent accumulation of ectopic fat or to reduce the amount of ectopic fat in the subject. In one instance, the amount of ectopic fat is reduced in a subject by at least about 5% compared to the subject prior to administration of the combination of anti-obesity agents provided that the anti-obesity agents are administered in amounts effective to reduce the body weight of the subject by at least 10%. In other instances, the amount of ectopic fat is reduced in a subject by at least about 10%, or by at least about 15%, 20%, 25%, 30% 40%, or 50%. Alternatively, the amount of ectopic fat is proportionally reduced 5%, 10%, 15%, 20%, 25%, 30%, 40%, 50%, 60%, 70%, 80%, 90%, or 100% in comparison to subcutaneous fat in a subject. Ectopic fat can be measured in a subject using any method available for measuring ectopic fat.

In another embodiment, uses are provided for producing a more favorable fat distribution in a subject, where to a subject a combination of anti-obesity agents is to be administered in amounts effective to produce a favorable fat distribution provided that the anti-obesity agents are administered in amounts effective to reduce the body weight of the subject by at least 10%. In one embodiment, administration of a combination of anti-obesity agents reduces the amount of visceral fat or ectopic fat, or both, in a subject. In one embodiment, the amount of visceral or ectopic fat, or a combination of both, preferentially reduced over the reduction in subcutaneous fat. This results in a higher ratio of subcutaneous fat to visceral fat or ectopic fat. Such improved ratios may result in a reduced risk of the development of cardiovascular diseases, polycystic ovary syndrome, metabolic syndrome, or any combinations thereof. In one embodiment, ectopic or visceral fat is metabolized at a rate 5% greater than subcutaneous fat. In other embodiments, ectopic or visceral fat is metabolized at a rate at least 10% 15%, 20%, 25%, 30% 50%, 60%, 70%, 80%, 90%, or 100% greater than subcutaneous fat.

In still another aspect, the therapeutically effective amount of a combination of anti-obesity agents is to be administered in combination with glucocortico steroids. Glucocortico steroids have the adverse effect of increasing fat mass and decreasing lean mass. Accordingly, it is contemplated that the anti-obesity agent combination can be used in conjunction with glucocortico steroids under conditions where glucocortico steroid use is beneficial.

Also provided are uses to reduce weight in a morbidly obese subject by first reducing the subject's weight to a level below that of being morbidly obese, then administering to the subject a combination of anti-obesity agents in effective amounts to further reduce the subject's weight. Methods for reducing a subject's weight to below that of morbid obesity include reducing caloric intake, increasing physical activity, drug therapy, bariatric surgery, such as gastric bypass surgery, or any combinations of the preceeding methods. In one aspect, administering the combination of anti-obesity agents further reduces the weight of the subject In another embodiment, use are provided for reducing the body mass index in a subject having a body mass index of 40 or less wherein the combination of anti-obesity agents is to be administered in effective amounts to further reduce the subject's weight provided that the anti-obesity agents are administered in amounts effective to reduce the body weight of the subject by at least 10%.

By reducing weight it is meant that the subject loses a portion of his/her total body weight over the course of treatment, whether the course of treatment be days, weeks, months or years. An effective amount of the anti-obesity agents administered in combination is an amount effective to reduce a subject's body weight over the course of the treatment, or alternatively an amount effective to reduce the subject's percentage of fat mass over the course of the treatment. The subject's body weight is reduced, over the course of treatment, by at least about 10%, by at least about 15%, or by at least about 20%. The subject's percentage of fat mass may be reduced, over the course of treatment, by at least 1%, at least 5%, at least 10%, at least 15%, at least 20%, or at least 25%.

In certain embodiments, reducing weight, in a subject, an effective amount of the anti-obesity agents is to be administered in a bolus dose one or more times a day. A bolus dose is an intermittent dosage of medicine (as opposed to a continuous infusion). A subject can be administered one or more bolus doses per day. The bolus dose can be the same no matter when it is administered to the subject, or can be adjusted such that the subject is administered a larger bolus dose at certain times of the day as compared to others. Administration of an agent in certain formulations, e.g., sustained-release formulations, a bolus dose can be administered less frequently, for example, once every three days, once per week, twice a month, once every month. Furthermore, the time between bolus doses is preferably long enough to allow the drug administered in the previous bolus dose to clear the subject's blood stream.

In other embodiments, for reducing weight, in a subject an effective amount of the anti-obesity agents in to be administered in continuous doses. By continuous dose it is intended to mean the continuous infusion of the drug by, for example, intravenous injection or a transdermal patch. Alternatively, a continuous dose can be administered orally in the form of a controlled release capsule or tablet which releases the drug into the subject's system over a period of time. When administered by a continuous dose, the drug is released over a period of about 1 hour, in some cases the drug is released over a period of about 2, 3, 4, 5, 6, 7, 8, 9, 10, 11, 12, 18, or 24 hours.

By "administered in combination" is meant that the anti-obesity agents are administered as a single administration, simultaneously as separate doses, or as sequentially administered. Sequential administration refers to administering one of the anti-obesity agents either before or after an anti-obesity agent. In one embodiment, the first anti-obesity agent is administered about 30 minutes before or after the at least one other anti-obesity agent, in other embodiments about 1, 2, 3, 4, 5, 6, 7, 8, 9, 10, 11, or 12 hours before or after the at least one other anti-obesity agents. Any of the administered anti-obesity agents can be administered as a bolus dose or as a continuous dose.

Herein are/is described is further directed to uses for increasing thermogenesis in a subject provided that the anti-obesity agents are administered in amounts effective to reduce the body weight of the subject by at least 10%, wherein a subject in need thereof is to be administered an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both. Thermogenesis is the process of liberating calories as heat by increasing the body's metabolic rate. Thermogenesis is activated by mechanisms, including supplements, nutrition, exercise, and exposure to cold.

Herein are/is described uses for increasing oxidative metabolism in a subject provided that the anti-obesity agents are administered in amounts effective to reduce the body weight of the subject by at least 10%, wherein a subject in need thereof is to be administered an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both. Oxidative metabolism is the process by which oxygen is used to make energy from carbohydrates (sugars).

In another aspect, uses for inducing a feeling of fullness in a subject are provided, provided that the anti-obesity agents are administered in amounts effective to reduce the body weight of the subject by at least 10% wherein an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject.

In yet another aspect, a use for controlling hunger in a subject is provided, provided that the anti-obesity agents are administered in amounts effective to reduce the body weight of the subject by at least 10% wherein an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject.

In yet a further aspect, a use for prolonging a feeling of satiation in a subject is provided, provided that the anti-obesity agents are administered in amounts effective to reduce the body weight of the subject by at least 10% wherein an effective amount of the at least one anti-obesity agent that acts upon forebrain structures involved in food intake, body weight modulation, or both in combination with administration of at least one anti-obesity agent that acts upon hindbrain structures involved in food intake, body weight modulation, or both is to be administered to said subject.

In yet a further aspect, a use for reducing caloric intake by reducing the size of a meal is provided, wherein an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject provided that the anti-obesity agents are administered in amounts effective to reduce the body weight of the subject by at least 10%.

In another aspect, a use for controlling food intake is provided, wherein the effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject provided that the anti-obesity agents are administered in amounts effective to reduce the body weight of the subject by at least 10%.

In yet another aspect, a use for ensuring or assisting in compliance with a reduced calorie or restrictive diet is provided, wherein an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject provided that the anti-obesity agents are administered in amounts effective to reduce the body weight of the subject by at least 10%.

In a further aspect, a use for adjusting a subject's set point so that the body's propensity for homeostasis is adjusted to a healthier set point is provided, wherein an effective amount of the at least one and-obesity agent that acts upon forebrain structures involved in food intake, body weight modulation, or both in combination with administration of the at least one anti-obesity agent that acts upon hindbrain structures involved in food intake, body weight modulation, or both is to be administered to said subject provided that the anti-obesity agents are administered in amounts effective to reduce the body weight of the subject by at least 10%.

In yet a further aspect, a use for maintaining weight loss or maintaining the weight lost is provided, wherein an effective amount of the at least one anti-obesity agent that acts upon forebrain structures involved in food intake, body weight modulation, or both in combination with administration of the at least one anti-obesity agent that acts upon hindbrain structures involved in food intake, body weight modulation, or both is to be administered to said subject. In one embodiment of this aspect of the invention, the weight loss is maintained by re-setting the subject's set point provided that the anti-obesity agents are administered in amounts effective to reduce the body weight of the subject by at least 10%.

Optionally, administration of the anti-obesity agents in combination results in a synergistic effect in any of the methods described herein. In addition, optionally, administration of the anti-obesity agents in combination results in a lower dosage requirement for at least one of the agents, with the same effect.

The teaching of the invention is of use in treating obesity in metabolic conditions or disorders that benefit from a reduction in nutrient availability. Accordingly, this teaching may be useful in the manufacture of a medicament for treating and/or preventing obesity, diabetes (e.g., type 2 or non-insulin dependent diabetes, type 1 diabetes, and gestational diabetes), eating disorders, insulin-resistance syndrome, and cardiovascular disease.

In one embodiment, a use for altering fat distribution, reducing fat mass, or both in a subject is provided provided that the anti-obesity agents are administered in amounts effective to reduce the body weight of the subject by at least 10%. Accordingly, subjects for whom altering body composition is of benefit can also benefit from the present invention. Altered body composition, as intended herein, includes loss or maintenance of body fat, with minimization of loss, maintenance, or gain of lean body mass. In such situations, weight may increase as well as decrease. Accordingly, subjects may be lean, overweight, or obese as these terms are generally used in the art. Uses of the invention may also include reducing fat in non-adipose tissue while sparing lean mass. Uses include treating diseases such as nonalcoholic steatohepatitis (NASH) or lipodystrophy.

Anti-obesity agents for use in the present invention include leptin, leptin derivatives, recombinant leptin, and leptin agonists. Leptin (derived from Greek *leptos,* meaning thin) is a hormone produced predominantly by fat cells. In obese humans, leptin blood levels generally correlate with the amount of fat stored in the body. Generally, the greater the amount of fat, the greater the amount of leptin. Serum leptin levels concentrations in the majority of humans with obesity are high, and a state of leptin resistance is thought to exist (Mantzoros et al. (2000) J. Clin. Endocrinol. Metab. 85:4000-4002). Despite therapeutic attempts at using leptin to treat obesity, the effect of recombinant human leptin has been limited, if any, in causing weight loss in obese individuals. Exceptions to this include the treatment of individuals with congentital leptin deficiency and the treatment of individuals with lipoatrophy. See, for example, Heymsfield et al. (1999) JAMA 282:1568-1575, Farooqi et al. (1999) N. Engl. J. Med. 341:879-884, and U.S. Pat. Publication No. 2005/0020496.

Serum leptin levels can be measured by methods known in the art, including, for example, using commercially available immunoassays.

It is one aspect of the invention that fat is reduced by means such as administration of amylin to treat leptin resistance. Once leptin resistance is ameliorated (lessened), leptin is administered to further treat obesity.

Leptin proteins and leptin protein containing compositions appropriate for uses described herein are known in the art and include, but are not limited to recombinant human leptin (PEG-OD, Hoffman La Roche) and recombinant methiony human leptin (Amgen). Leptin proteins, analogs, derivatives, preparations, formulations, pharmaceutical compositions, doses, and administration routes have previously been described in the following patent publications U.S. Pat. Nos. 5,552,524; 5,552,523; 5,552,522; 5,521,283; and PCT Application Publication Nos. WO 96/05309, WO 96/40912; WO 97/06816, WO 00/20872, WO 97/18833, WO 97/38014, WO 98/08512, and WO 98/284427.

Leptin agonists and antagonists are known in the art and can also be found through a search of the patent databases. For example, leptin agonists are described in U.S. Pat. Publication Nos. 2004/0072219, 2003/049693, 2003/0166847, 2003/0092126, and U.S. Pat. Nos. 6,777,388 and 6,936,439. Leptin antagonists are described in U.S. Pat. Publication Nos. 2004/0048773, 2002/0160935 and U.S. Pat. No. 6,399,745. Means for testing for leptin agonism or antagonism are described in U.S. Pat. Nos. 6,007,998 and 5,856,098. These patent are exemplary.

Anti-obesity agents for use in the present invention also include amylin and amylin agonists having at least 80 % amino acid sequence identity to SEQ ID NO: 1 having amylin activity. Amylin is a 37 amino acid peptide hormone that is co-secreted with insulin from pancreatic beta-cells in response to nutrient stimuli. Human amylin (hAmylin) has the following amino acid sequence:
Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr (SEQ ID NO:1). Rat amylin (rAmylin) has the following sequence:
KCNTATCATQRLANFLVRSSNNLGPVLPPTNVGSNTY (SEQ ID NO:2). The use of amylins from any species is contemplated.

It has surprisingly been found that modulation of effective amylin levels in vivo such as through the use of amylin, amylin agonists, and amylin antagonists, can modulate the effective levels of ghrelin in vivo.

Amylin agonists having at least 80 % amino acid sequence identity to SEQ ID NO: 1 having amylin activity. contemplated in the use of the invention include those described in U.S. Patent Nos. 5,686,411, 6,114,304, and 6,410,511. Such compounds include those having the formula I:
¹A₁-X-Asn-Thr-⁵Ala-Thr-Y-Ala-Thr-¹⁰Gln-Arg-Leu-B₁-Asn-¹⁵Phe-Leu-C₁-D₁-E₁-²⁰F₁-G₁-Asn-H₁-Gly-²⁵I₁-J₁-Leu-K₁-L₁-³⁰-Thr-M₁-Val-Gly-Ser-³⁵Asn-Thr-Tyr-Z (SEQ ID NO:3)
wherein A₁ is Lys, Ala, Ser or hydrogen;
B, is Ala, Ser or Thr;
C₁ is Val, Leu or Ile;
D₁ is His or Arg;
E₁ is Ser or Thr;
F₁ is Ser, Thr, Gln or Asn;
G₁ is Asn, Gln or His;
H₁ is Phe, Leu or Tyr;
I₁ is Ala or Pro;
J₁ is Ile, Val, Ala or Leu;
K₁ is Ser, Pro, Leu, Ile or Thr;
L₁ is Ser, Pro or Thr;
M₁ is Asn, Asp, or Gln;
X and Y are independently selected amino acid residues having side chains which are chemically bonded to each other to form an intramolecular linkage; and
Z is amino, alkylamino, dialkylamino, cycloalkylamino, arylamino, aralkylamino, alkyloxy, aryloxy or aralkyloxy and having at least 80% amino acid sequence identity to SEQ ID NO : 1 having amylin activity.

Suitable side chains for X and Y include groups derived from alkyl sulthydryls which may form disulfide bonds; alkyl acids and alkyl amines which may form cyclic lactams; alkyl aldehydes or alkyl halides and alkylamines which may condense and be reduced to form an alkyl amine bridge; or side chains which may be connected to form an alkyl, alkenyl, alkynyl, ether or thioether bond. Preferred alkyl chains include lower alkyl groups having from about 1 to about 6 carbon atoms.

An additional aspect of the present invention is directed to agonist analogues of SEQ ID NO:3 which are not bridged, and wherein X and Y are independently selected from Ala, Ser, Cys, Val, Leu and Ile or alkyl, aryl, or aralkyl esters and ethers of Ser or Cys.

Biologically active derivatives of the above agonist analogs are also included within the scope of this invention in which the stereochemistry of individual amino acids may be inverted from (L)/S to (D)/R at one or more specific sites.

Also included within the scope of this invention are the agonist analogs modified by glycosylation of Asn, Ser and/or Thr residues.

Biologically active agonist analogs of amylin are included within the scope of this invention which contain less peptide character. Such peptide mimetics may include, for example, one or more of the following substitutions for --CO-NH-- amide bonds: depsipeptides (-CO-O-), iminomethylenes (-CH2 -NH-), trans-alkenes (-CH=CH-), beta-enaminonitriles (--C(=CH-CN)--NH--), thioamides (-CS--NH-), thiomethylenes (-S--CH2 -- or -CH2 --S--), methylenes (--CH2 --C2 --) and retro-amides (--NH--CO--).

Compounds used in this invention form salts with various inorganic and organic acids and bases. Such salts include salts prepared with organic and inorganic acids, for example, HCl, HBr, H₂SO₄, H₃PO₄, trifluoroacetic acid, acetic acid, formic acid, methanesulfonic acid, toluenesulfonic acid, maleic acid, fumaric acid and camphorsulfonic acid. Salts prepared with bases include, for example, ammonium salts, alkali metal salts (such as sodium and potassium salts) and alkali earth salts (such as calcium and magnesium salts). Acetate, hydrochloride, and trifluoroacetate salts are preferred.

Throughout the application, the amino acid sequences may be referred to as amino acids at position a to position b adjacent to a reference peptide. For example, 1-7 hAmylin refers to the amino acid sequence from position 1 to position 7, inclusive, of human amylin (SEQ ID NO:1), the reference peptide in this example. Modification to the reference peptide may be shown as the position of modification adjacent to the modification. For example, (2Asp 7Lys) 1-7 hAmylin represents the amino acid sequence at positions 1 to 7 of human amylin with a modification of the Cys to Asp at position 2 and a modification of the Cys to Lys at position 7. For another example, ¹⁸Arg^{25,28}Pro-h-amylin represents the amino acid sequence of human amylin with a modification of the His to Arg at position 18, a modification of the Ala to Pro at position 25, and a modification of the Ser to Pro at position 28.

Exemplary compounds include, but are not limited to des-¹Lys-h-amylin (SEQ ID NO:4), ²⁸Pro-h-amylin (SEQ ID NO: 5), ^{25,28,29}Pro-h-amylin (SEQ ID NO:6), ¹⁸Arg^{25,28}Pro-h-amylin (SEQ ID NO:7), and des-¹Lys¹⁸Arg^{25,28}Pro-h-amylin (SEQ ID NO: 8), all show amylin activity in vivo in treated test animals, (e.g., provoking marked hyperlactemia followed by hyperglycemia). In addition to having activities characteristic of amylin, certain of the preferred compounds of the invention have also been found to possess more desirable solubility and stability characteristics when compared to human amylin. Examples of these compounds include ²⁵Pro²⁶Val^{28,29} Pro-h-amylin (SEQ ID NO:9), ^{25,28,29}Pro-h-amylin (SEQ ID NO:10), and ¹⁸Arg^{25,28}Pro-h-amylin (SEQ ID NO:7).

Other compounds include ¹⁸Arg^{25,28,29}Pro-h-amylin (SEQ ID NO:11), des- ¹Lys¹⁸Arg^{25,28,29}Pro-h-amylin (SEQ ID NO: 12), des-¹ Lys^{25,28,29}Pro-h-amylin (SEQ ID NO:13), ²⁵Pro²⁶-Val^{28,29}Pro-h-amylin (SEQ ID NO: 14), ²³Leu²⁵Pro²⁶Val^{28,29}Pro-h-amylin (SEQ ID NO:15), ²³Leu²⁵Pro²⁶Val²⁸Pro-h-amylin (SEQ ID NO:16), des- ¹Lys²³Leu²⁵Val²⁶Val²⁸Pro-h-amylin (SEQ ID NO:17), ¹⁸Arg²³Leu²⁵Pro²⁶Val²⁸Pro-h-amylin (SEQ ID NO:18), ¹⁸Arg²³Leu^{25,28,29}Pro-h-amylin (SEQ ID NO:19), ¹⁸Arg²³Leu^{25,28}Pro-h-amylin (SEQ ID NO:20), ¹⁷Ile²³Leu^{25,28,29}Pro-h-amylin (SEQ ID NO:21), ¹⁷Ile^{25,28,29}Pro-h-amylin (SEQ ID NO:22), des-¹Lys¹⁷Ile²³Leu^{25,28,29}Pro-h-amylin (SEQ ID NO:23), ¹⁷Ile¹⁸Arg²³Leu-h-amylin (SEQ ID NO:24), ¹⁷Ile¹⁸Arg²³Leu²⁶Val²⁹Pro-h-amylin (SEQ ID NO:25), ¹⁷Ile¹⁸Arg²³Leu²⁵Pro²⁶Val^{28,29}Pro-h-amylin (SEQ ID NO:26), ¹³Thr²¹His²³Leu²⁶Ala²⁸Leu²⁹Pro³¹ Asp-h-amylin (SEQ ID NO:27), ¹³Thr²¹His²³Leu²⁶Ala²⁹Pro³¹Asp-h-amylin (SEQ ID NO:28), des-¹Lys¹³Thr²¹His²³Leu²⁶Ala²⁸Pro³¹Asp-h-amylin (SEQ ID NO:29), ¹³Thr¹⁸ Arg²¹-His²³Leu²⁶Ala²⁹Pro³¹ Asp-h-amylin (SEQ ID NO:30), ¹³Thr¹⁸Arg²¹His²³Leu^{28,29}Pro³¹ASp-h-amylin (SEQ ID NO:31), and ¹³Thr¹⁸Arg²¹His²³Leu²⁵Pro²⁶Ala^{28,29}Pro³¹ Asp-h-amylin (SEQ ID NO:32).

Useful amylin agonist analogs having at least 80 % amino acid sequence identity to SEQ ID NO:1 having amylin activity include those identified in PCT Application Publication No. WO 93/10146.

Amylin agonists useful in the invention may also include fragments of amylin and its analogs having at least 80 % amino acid sequence identity to SEQ ID NO : 1 having amylin activity as described above as well as those described in EP 289287. Amylin agonists are compounds having at least 80, 85, 90, 95, or 99% amino acid sequence identity to SEQ ID NO:1 having amylin activity. "Amylin activity" as used herein includes the ability of amylin to affect ghrelin levels in a body. Amylin agonists also include analogs of amylin having insertions, deletions, extensions and/or substitutions in at least one or more amino acid positions of SEQ ID NO:1 that have at least 80 % amino acid sequence identity to SEQ ID NO: 1 having amylin activity. The number of amino acid insertions, deletions, or substitutions may be not more than 5, 10, 15, 20, 25, or 30. Insertions, extensions, or substitutions may be with other natural amino acids, synthetic amino acids, peptidomimetics, or other chemical compounds.

Amylin agonists also include polypeptides (referred to herein as LHC (loop helix C-terminus) peptides) described in U.S. Patent Application No. 60/543,275 and in PCT application No. PCT/US2005/004631, filed Febrary 11, 2005, as well as their analogs and derivatives. The LHC peptides for use in the invention act as an agonist for at least one biological effect of calcitonin, amylin, CGRP, or any combination of the three herein disclosed or bind to at least one of the receptors of amylin, calcitonin, or CGRP. Receptor binding activity and biological activity of exemplary LHC peptides are described in U.S. Patent Application No. 60/543,275 and in PCT application No. PCT/US2005/004631. In a general aspect, these polypeptide agonists have at least a loop region of amylin or calcitonin and analogs thereof, an α helix region of at least a portion of an α helix region of calcitonin or analogs thereof or an α helix region having a portion of an amylin a helix region and a calcitonin α helix region or their respective analogs, and a C-terminal tail of amylin or calcitonin or analogs thereof, with the proviso that the C-terminal tail of calcitonin or a calcitonin analog is not proline (Pro), hydroxyproline (Hyp), homoserine (Hse) or derivatives of Hse.

In certain embodiments, these LHC peptides have an amylin or amylin analog loop region, at least a portion of a calcitonin or calcitonin analog α helix region, and an amylin or amylin analog C-terminal tail. In still other embodiments, these LHC peptides have an amylin or amylin analog loop region, at least a portion of an amylin or amylin analog α helix region and at least a portion of a calcitonin or calcitonin analog α helix region, and an amylin or amylin analog C-terminal tail. In yet other embodiments, these LHC peptides have a calcitonin or calcitonin analog loop region, at least a portion of an amylin or amylin analog a helix region and at least a portion of a calcitonin or calcitonin analog α helix region, and an amylin or amylin analog C-terminal tail. In still yet other embodiments, these LHC peptides have an amylin or amylin analog loop region, a portion or a calcitonin or calcitonin analog α helix region or at least a portion of an amylin or amylin analog α helix region and at least a portion of a calcitonin or calcitonin analog α helix region, and a calcitonin or calcitonin analog C-terminal tail.

In certain embodiments, the loop region of these LHC peptides may further comprise no more than one, two, three, or four modifications including substitutions, insertions, or deletions from the amylin or calcitonin loop, and analogs thereof. It is further contemplated that these LHC peptides may have additional modifications at the N-terminal portion of the loop comprising a N-cap region, that may have hydrophobic or hydrophilic characteristics such as acetyl, isocaproyl, 3,6-dioxyoctanoic acid, or 1-amino-4,7,10-trioxa-13-tridecanamine succinimic acid. Modifications may further include one, two, three or more additional amino acids. This is an area which allows for many modifications too numerous to mention, but would be understood by one of skill in the art based upon what is exemplified further in the present application.

These LHC peptides may also be further derivatized by chemical alterations such as amidation, glycosylation, acylation, sulfation, phosphorylation, acetylation, and cyclization. Such chemical alterations may be obtained through chemical or biochemical methodologies, as well as through *in vivo* processes, or any combination thereof. Derivatives of these LHC peptides may also include conjugation to one or more polymers or small molecule substituents. One type of polymer conjugation is linkage or attachment of polyethylene glycol ("PEG'') polymers, polyamino acids (*e.g.,* poly-his, poly-arg, poly-lys, etc.) and/or fatty acid chains of various lengths to the N- or C-terminus or amino acid residue side chains of the polypeptide. Small molecule substituents include short alkyls and constrained alkyls (e.g., branched, cyclic, fused, adamantyl), and aromatic groups. In addition, basic residues such as R and K may be replaced with homoR and homoK, citrulline, or ornithine to improve metabolic stability of the peptide. Polypeptides for use in the invention include acid as well as amide forms.

In certain embodiments, the α helix region of the LHC peptides comprise at least four consecutive amino acids of a calcitonin or calcitonin analog a helix region. In other embodiment, the α helix region comprises at least 5, 6, 7, or 8 consecutive amino acids of a calcitonin or calcitonin analog α helix region. In certain embodiments, when the number of consecutive amino acids are less than 8, it is contemplated that the a helix region further comprises at least 4, 5, 6, 7, 9, 10, 11, or more consecutive amino acids of an amylin or amylin analog a helix region. In certain embodiments, it is envisioned that the less amino acids of calcitonin or calcitonin analog, the more amino acids of an amylin or amylin analog may be found in the α helix region of the novel compounds. The number of amino acids comprising the α helix region may be from about 10 to 23 amino acids. Accordingly, the α helix region may be 10,11, 12, 13, 14,15, 16, 17, 18, 19, 20, 21, 22, or 23 amino acids long. Moreover, the amino acids should provide for about three to about six α helical turns. It is further contemplated that the a helix region of the compounds may further comprise no more than one, two, three, four, five, six, seven, eight, nine or ten modifications including substitutions, insertions, or deletions from that of the calcitonin, and/or amylin α helix region, and analogs thereof.

Herein are/is described, the C-terminal tail of the LHC peptides comprise at least the last six, five, or four amino acids of either amylin or calcitonin, and analogs thereof. Herein are/is described, the C-terminal tail of the novel compounds comprise at least a portion of the C-terminal end having a β turn. Herein are/is described, the β turn is introduced by the amino acid combination of Gly-Ser. Accordingly, the LHC peptides may have a C-terminal end comprising a portion of an amylin or calcitonin C-terminal tail (and analogs thereof) having Gly-Ser or starting at Gly-Ser.

Herein are/is described, the C-terminal tail of the LHC peptides may further comprise no more than one, two, or three, modifications including substitutions, insertions, or deletions from the amylin or calcitonin loop, and analogs thereof. It is further contemplated that the LHC peptides may have additional modifications at the C-terminal portion of the C-terminal tail which may include, for example, L-octylglycine, 4ABU (4-aminobutyric acid), 9Anc (9 amiononanoic acid), 3,6-dioxyoctanoic acid or 1-amino-4,7,10-trioxa-13-tridecanamine succinimic acid. Modification may further include one, two, three or more additional amino acids. The types of modification contemplated in this area would be understood by one of skill in the art based upon what is exemplified further in the present application.

In one aspect, a loop region is defined as that region found at the N-terminal end comprising at least 5 to 8 amino acids, wherein the first and last amino acid are capable of creating a bond, for example, residues at positions 2-7 of amylin or residues at positions 1-7 of calcitonin and their corresponding regions in their respective analogs. In another aspect, a a helix region is defined as the internal portion of amylin or calcitonin flanked by the loop region and the C-terminal tail which structurally forms an α helix, for example, residues at positions 8-23 of amylin or residues at positions 8-27 of calcitonin and their corresponding regions in their respective analogs. In yet another aspect, a C-terminal tail is defined as that region after the a helix, *e.g*., residues at positions 33-37 of amylin or longer such as residues at positions 27-37 or residues at positions 27 or 28 to 32 of calcitonin. Included in the LHC peptides are both the amide and acid forms of the disclosed compounds.

Amylin and calcitonin, as herein defined, includes all native and species variations. Examples of amylin and calcitonin include, but are not limited to: human amylin (SEQ ID NO:1), rat amylin (SEQ ID NO:2), salmon calcitonin (sCT) CSNLSTCVLGKLSQELHKLQTYPRTNTGSGTP (SEQ ID NO:33), and human calcitonin (hCT) CGNLSTCMLGTYTQDFNKFHTFPQTAIGVGAP (SEQ ID NO:34).

In a general aspect, the LHC peptides comprise at least a loop region, an a helix region, and a C-terminal tail. The loop region comprises an amino sequence comprising the formula (II) X-Xaal sequence-Y wherein X and Y are capable of creating a bond and are independently selected residues having side chains which are, or are capable of being, chemically bonded to each other to form an intramolecular linkage such as, for example, a disulfide bond; an amide bond; a cyclic lactam formed, for example, by an alkyl acid and an alkyl amine; an alkyl amine or imine bridge formed, for example, by condensing and reducing an alkyl aldehydes or alkyl halides and alkylamines; and an alkyl, alkenyl, alkynyl, ether or thioether bond formed, for example, by connection of side chains. Alkyl chains may include lower alkyl groups having from about 1 to about 6 carbon atoms. Herein are/is described, the intramolecular linkage may be a disulfide, amide, imine, amine, alkyl and alkene bond. Herein are/is described, X and Y of formula (II) are independently selected from Ser, Asp, Glu, Lys, Orn (ornithine), or Cys. Herein are/is described, X and Y of formula (II) are Cys and Cys. Herein are/is described, X and Y of formula (II) are Ser and Ser. In still other embodiments, X and Y of formula (II) are Asp and Lys or Lys and Asp.

The Xaa1 sequence of formula (II) comprises an amino acid sequence of 3, 4, 5, or 6 amino acids between X and Y. Herein are/is described, the Xaa1 sequence comprises an amino acid sequence having a region with one or more substituted or unsubstituted hydroxyl-containing residues next to Y. For example, the hydroxyl containing residue region may have at least 2 of the 3 amino acids adjacent Y that are either a Ser or Thr. The other amino acids in the Xaa1 sequence may be any amino acid. Herein are/is described, the Xaa1 sequence is 3 amino acids. In other embodiments, the Xaa1 sequence is 4 amino acids. Herein are/is described, the Xaa1 sequence is 5 amino acids. In yet other embodiments, the Xaa1 sequence is 6 amino acids. Accordingly, Xaa1 of formula (II) can be represented by Xaa2-Xaa3-Xaa4-Xaa5-Xaa6-Xaa7 (SEQ ID NO:35). Herein are/is described, Xaa2, Xaa3, Xaa4, any two, or all three may absent. Herein are/is described, Xaa5, Xaa6, and Xaa7 comprise the hydroxy-containing residue region. As such, at least two of the three amino acids can be a Ser, Hse, Thr, allo-Threonine (alloThr), d-Threonine (d-Thr), or other unnatural analog thereof. Xaa2 can be any amino acid or absent, Xaa3 can be any amino acid or absent, Xaa4 can be any amino acid or absent, Xaa5 can be any amino acid if Xaa6 is a Ser or Thr and Xaa7 is a Ser or Thr, Xaa6 can be any amino acid if Xaa5 is a Ser or Thr and Xaa7 is a Ser or Thr, Xaa7 can be any amino acid if Xaa5 is Ser or Thr and Xaa6 is Ser or Thr. Accordingly, Herein are/is described, Xaa1 can be represented as Xaa2 absent, Xaa3 is Ala, Gly, Ser, Asp or absent, Xaa4 is Asn, Ala, Asp, Gly or absent; Xaa5 is Ala, Leu, Thr, or Ser; Xaa6 is Ala, Ser, or Thr; and Xaa7 is Ala, Ser, Val, Hse, (S)-2-amio-3-hydroxy-methylbutanoic acid (Ahb), (2S,3R)-2-amino-3hydroxy-methylpentanoic acid (Ahp), d-Thr, Thr, or a derivative thereof. Herein are/is described Xaa1 can be represented as Xaa2 is absent, Xaa3 is Ser, Gly, or absent, Xaa4 is Asn or Asp, Xaa5 is Ala, Ser, Thr or Leu, Xaa6 is Ala, Thr or Ser, and Xaa7 is Ser, d-Thr, alloThr or Thr. Herein are/is described, the loop region of formula (II) comprises the above-described representations of Xaa1 wherein Xaa3 is Ala, wherein Xaa3 is Ser or wherein Xaa3 is Gly. Alternatively or additionally, the loop region comprises the above described representations of Xaa1 wherein Xaa4 is Ala, wherein Xaa4 is Asn, wherein Xaa4 is Asp, or wherein Xaa4 is Gly. Alternatively or additionally, the loop region comprises the above-described representations of Xaa1 wherein Xaa5 is Ala, wherein Xaa5 is Thr, or wherein Xaa5 is Leu. Alternatively or additionally, the loop region comprises the above described representations of Xaa1 wherein Xaa6 is Ser or wherein Xaa6 is Ala. Alternatively or additionally, the loop region comprises the above-described representations of Xaa1 wherein Xaa7 is Thr or wherein Xaa7 is d-Thr. It is further contemplated that no more than one, two, or three modifications such as substitutions, insertions, deletions, and/or derivatizations may be made to the loop region.

Examples of the loop region include, but are not limited to, CNTATC (SEQ ID NO:36); CATATC (SEQ ID NO:37); CDTATC (SEQ ID NO:38); CGTATC (SEQ ID NO:39); CNAATC (SEQ ID NO:40); CNTSTC (SEQ ID NO:41); CNTA-dThr-C (SEQ ID NO:42); CNTA-T(OPO₃H₂)-C (SEQ ID NO:43); CNTASC (SEQ ID NO:44); CNTAAC (SEQ ID NO:45); CNTAVC (SEQ ID NO:46); CNTA-Hse-C (SEQ ID NO:47); CNTA-Ahb-C (SEQ ID NO:48); CNTA-Ahp-C (SEQ ID NO:49); CSNLSTC (SEQ ID NO:50); CGNLSTC (SEQ ID NO:51); CANLSTC (SEQ ID NO:52); CSALSTC (SEQ ID NO:53); CSNASTC (SEQ ID NO:54); CSNLATC (SEQ ID NO:55); and CSNLSAC (SEQ ID NO:56). As previously noted, it is further contemplated that no more than one, two, or three modifications such as substitutions, insertions, deletions, and/or derivatizations may be made to the loop region.

The loop region of the LHC peptides may further comprise modifications or additional amino acids at the N-terminal end. Such modifications include the addition of compounds such as Lys, Ala, Phe, Ile, Ser, Octylglycine, Isocap, Fmoc-3,6-dioxyoctanoic acid, Fmoc-1-amino-4,7,10-trioxa-13-tridecanamine succinimic acid, acetyl, and/or groups for solubility, delivery, signaling. Exemplary modified loops include the addition of Lys to the sequence of Xaa1 or the addition of Ile to the sequence of Xaa1. For example, the modified loop region may be KCNTATC (SEQ ID NO:57). In certain embodiments, the additions and/or modifications at the N-terminal end of the loop region may change the loop region. For example, the loop region may be modified as follows: cyclo(2,7) 1-7 hAmylin, cyclo(²Asp⁷Lys) 1-7 hAmylin, N-isocaproyl 1-7 hAmylin, N-3,6 dioxaoctanoyl 1-7 hAmylin, L-Octylglycine 1-7 hAmylin, Acetyl (²Agy, ⁷Agy) 1-7 hAmylin wherein Agy is Allylglycine, Acetyl (¹Ala) 1-7 hAmylin, (¹Thr ³Asp) 1-7 hAmylin, Isocap (⁷Ala) 5-7 sCT, Acetyl (²Agy, ⁷Agy) 1-7 sCT, and cyclo (1,7 (¹Asp ⁷Lys) 1-7 sCT. Therefore, taking the example of Isocap (⁷Ala) 5-7 sCT, herein are/is described modifications at the N-terminal region of the loop region such that amino acids Xaa2 to Xaa5 are absent.

The a helix region of the LHC peptides may be about 8 to 23 amino acids in length. Herein are/is described, the α helix region is amphiphatic. Herein are/is described, the a helix region comprises about 3 to 6 helical turns. Herein are/is described, the a helix region comprises 3, 4, 5, or 6 helical turns. Herein are/is described, the a helix region is a rigid structure equivalent to about 3, 4, 5, or 6 helical turns. Herein are/is described, the α helix is an ampliphatic structure. Accordingly, characteristics of desirable amino acids that would provide this type of structure may be selected.

It has been found that the calcitonin α helix region, a combination of an amylin and a calcitonin α helix region, or parts thereof, and/or some CGRP elements are desirable in the α helix region of the LHC peptides. It is contemplated that, as with the loop region, the α helix region can be from any amylin or calcitonin, and analogs thereof. Accordingly, herein are/is described, the a helix region is at least a portion of an α helix region of a calcitonin or calcitonin analog. Herein are/is described, the α helix region is at least a portion of an a helix region of a calcitonin or calcitonin analog and at least a portion of an a helix of an amylin or amylin analog. Herein are/is described, the α helix region of the LHC peptides contain elements of CGRP. It is further contemplated that novel compounds may have no more than one, two, three, four, five, six, seven, eight, nine, or ten further modifications such as substitutions, insertions, deletions, and/or derivatizations.

Herein are/is described, the a helix region of the LHC peptides may comprise a helix region type II. An α helix region type II comprises a portion of an a helix region of an amylin or amylin analog and a portion of an α helix region of a calcitonin or calcitonin analog. The a helix region type II may comprise amino acids from position 8 ofhAmylin to 11, 12, 13,14, 15, 16, 17, 18 or 19 ofhAmylin and amino acids from position 13, 14, 15, 16, 17,18, and 19 of sCT to position 18,19, 20, 21, 22, 23, 24, 25, 26, or 27 of sCT. Alternatively or additionally, the above described α helix region of amylin and calcitonin may further comprise the substitutions of one or more of (⁸Val), (⁹Leu), (⁹Met), (¹⁰Gly), (¹⁰His), (¹²Thr), (¹³Thr), (¹³Asn), (¹³Phe), (¹³Tyr), (¹⁴Arg), (¹⁴Ala), (¹⁴Asp), (¹⁴Glu), (¹⁴Gln), (¹⁴Thr), (¹⁴Gly), (¹⁵Leu), (¹⁵Ser), (¹⁵Glu), (¹⁵Ala), (¹⁵Tyr), (¹⁶Asp), (¹⁷Ser), (¹⁷Phe), (¹⁸Arg). (¹⁷Aib), (¹⁸Arg), (¹⁸Orn), (¹⁸hArg), (¹⁸Cit), (¹⁸hLys), (¹⁸Lys(for)), (¹⁸Lys(PEG5000)), (¹⁹Phe), (²⁰His), (²¹Asn), (²²Met), (²²Val), (²²Phe), (²²Leu), (²⁴Pro), or any combination thereof. Herein are/is described, the number of amino acids in the α helix region type II of the LHC peptides is at least 10 amino acids. Herein are/is described, the number of amino acids in the a helix region type IT of the LHC peptides is 11,12, 13,14, 15, 16, 17, 18, 19, 20, 21, 22, or 23. Herein are/is described, the number of amino acids in the a helix region type II of the LHC peptides is 24 or more.

Herein are/is described, an α helix region type II of the LHC peptides can be represented by: Xl Xaa8 Xaa9 Xaa10 R Xaa12 Xaa13 Xaa14 Xaa15 Xaa16 Xaa17 Xaa18 Xaa19 Xaa20 Xaa21 Xaa22 P Xaa24 T N T Xl (SEQ ED NO:60) wherein
Xaa8 is Ala or Val;
Xaa9 is Thr, Met or Leu;
Xaa10 is Gln, Gly, His;
Xaa12 is Leu, or Thr,
Xaa13 is Ala, Thr, Asn, Phe, Tyr, Ser, or Thr;
Xaa14 is Asn, Arg, Ala, Asp. Glu, Gln, Thr, or Gly;
Xaa15 is Phe, Leu, Ser, Glu, Ala, Asp, or Tyr;
Xaa16 is Leu or Asp;
Xaa17 is Val, His, Ser, Phe, or Aib;
Xaa18 is His, Arg, Lys, Orn, hArg, Cit, hLys, Lys(for), or Lys(PEG5000);
Xaa19 is Leu, Ser or Phe;
Xaa20 is Gln or His;
Xaa21 is Thr or Asn;
Xaa22 is Tyr, Val, Phe,Leu or Met;
Xaa24 is Arg or Pro; and
Xl is absent or comprises 1-4 additional amino acids.

Again, each member in the Markush group, or a combination thereof, herein are/is described of the invention and is not to be read as a single unit. It is further contemplated that the α helix region type II may contain no more than one, two, three, four, five, six, seven, eight, nine, or ten modifications such as substitutions, insertions, deletions, and/or derivatizations of the compounds described herein. For example, herein are/is described, the α helix region type II may have deletions at the C-terminal end resulting in the deletion of position 27, 26, 25, 24, or 22. Herein are/is described, however, the deletions do not remove amino acids of positions 19,20,21, or 22.

Examples of an α helix region of type II of the LHC peptides include, but are not limited to, (⁸Val ⁹Leu ¹⁰Gly) 11-15 hAmylin 16-27 sCT, (⁸Val¹⁹Leu ¹⁰Gly) 11-15 hAmylin (¹⁸Arg) 16-27 sCT, 8-12 hAmylin (¹⁸Arg) 13-27 sCT, 8-18 hAmylin 19-23 sCT, 8-18 Amylin 19-27 sCT, (¹⁵Glu, ¹⁸Arg) 8-18 hAmylin 19-24 sCT, (¹⁴Arg ¹⁵Ser) 8-18 hAmylin 19-22 sCT, (¹³Ala ¹⁴Ala ¹⁵Ala) 8-18 hAmylin 19-27 sCT, (¹³Ala ¹⁴Asp ¹⁵Ala) 8-18 hAmylin 19-22 sCT, (¹³Ala ¹⁴Asp) 8-18 hAmylin 19-23 sCT, (¹³Ala ¹⁴Asp) 8-18 hAmylin 19-27 sCT, (¹³Ala ¹⁴Ala) 8-18 hAmylin 19-22 sCT, (¹³Ala ¹⁴Glu) 8-18 hAmylin 19-22 sCT, (¹³Thr ¹⁴Asp ¹⁵Tyr) 8-18 hAmylin 19-22 sCT, (¹³Ala ¹⁴Gln) 8-18 hAmylin 19-22 sCT, (¹³Asn ¹⁴Glu ¹⁵Tyr) 8-18 hAmylin 19-27 sCT, (¹³Phe ¹⁴Asp) 8-18 hAmylin 19-27 sCT, (¹³Ala ¹⁴Asp) 8-18 hAmylin (¹⁵Glu ¹⁸Arg) 8-18 hAmylin 19-24 sCT, (¹⁹Phe ²²Phe) 19-27 sCT, (¹³Ala ¹⁴Asp) 8-18 hAmylin (¹⁹phe ²²His ²²Phe) 19-27 sCT, (¹³Ala ¹⁴Asp) 8-18 hAmylin (¹⁹Phe ²²Phe) 19-27 sCT, (⁹Thr ¹⁰His) 8-18 hAmylin 19-22 sCT, (⁹Thr ¹⁰His ¹⁴Gly ¹⁵Leu ¹⁷Ser ¹⁸Arg) 8-19 hAmylin 20-23 sCT, 8-18 hAmylin (²¹Asn ²²Phe ²³Val) 19-23 sCT, 8-18 hAmylin (²²Met) 19-27 sCT, 8-18 hAmylin (²²Val) 19-27 sCT, (⁹Met ¹²Thr ¹³Tyr ¹⁴Thr ¹⁵ Glu ¹⁶Asp ¹⁷Phe) 8-17 hAmylin (¹⁸Arg) 18-20 sCT). Herein are/is described, novel compounds include variations of the above exemplary compounds with the a helix terminating at-corresponding to 22, 23, 24, 25, 26 or 27 of sCT. In other words, compound 8-18 hAmylin 19-24 sCT is also specifically described as this compound is merely 8-18 hAmylin 19-27-sCT described above truncated to position 24. As another example, compound (13Ala 14Asp, 15ala) 8-18 hAmylin 19-23 is specifically described because of the above language applied to (13Ala 14Asp 15Ala) 8-18 hAmylin 19-22.

Herein are/is described, the C-terminal tail of the LHC peptides comprises amino acids from position 27, 28, 29, 30, 31, 32, or 33 to position 36 or 37 ofhAmylin. Herein are/is described, the C-terminal tail of the LHC peptides comprises amino acids from position 27 or 28 to position 32 of sCT; however, when the loop region is from a calcitonin or calcitonin analog and the α helix region is from a calcitonin or calcitonin analog, the last position of the C-terminal tail is not Pro, Hyp, Hse or derivatives of Hse. Alternatively or additionally, the above described a helix of amylin and calcitonin may further comprise the substitutions of one or more of (²⁷Tyr) hAmylin, (²⁹Arg) hAmylin, (³²Val) hAmylin, (³²Thr) hAmylin, (³⁴Glu) hAmylin, (³⁵Lys) hAmylin, (³⁶Phe) hAmylin, (³⁶Ala) hAmylin, (³⁷Phe) hAmylin, (³⁰Asn) sCT, (³²Tyr) sCT, or any combination thereof.

Herein are/is described, a C-terminal tail of the LHC peptides can be represented by Xaa28 Xaa29 Xaa30 Xaa31 Xaa32 Xaa33 G Xaa35 Xaa36 Xaa37 Xaa38 (SEQ ID NO:61), wherein
Xaa28 is Lys, Tyr, or absent;
Xaa29 is Ser, Pro, or absent;
Xaa30 is Ser, Pro, Arg, or absent;
Xaa31 is Thr, or absent;
Xaa32 is Asn or absent;
Xaa33 is Val, Thr, or absent;
Xaa35 is Ser, Glu
Xaa36 is Asn, Lys, or Gly;
Xaa37 is Thr, Phe, or Ala;
Xaa38 is Tyr, Phe, Pro, or absent;
with the proviso that when the loop region of the LHC agonist is from a calcitonin or calcitonin analog and the α helix region is from a calcitonin or calcitonin analog, the last position of the C-terminal tail is not Pro, Hyp, Hse or derivatives of Hse.

Again, each member of the Markush group, or a combination thereof, herein are/is described and is not to be read as a single unit. It is further contemplated that the C-terminal tail may contain no more than one, two, or three modifications such as substitutions, insertions, deletions, and/or derivatizations of the compounds described herein.

Examples of the C-terminal tail of an LHC agonist include, but are not limited to, 27-37 rAmylin, (²⁷ Tyr ²⁹Arg ³²Thr) 27-37 rAmylin, (²⁹Arg ³²Thr) 28-37 rAmylin, 30-37 hAmylin, (³²Thr) 30-37 hAmylin, (³⁵Lys ³⁶Ala ³⁷Phe) 30-37 hAmylin, 30-36 hAmylin, (³²Val) 30-36 hAmylin, (³⁴Glu ³⁶Phe) 30-36 hAmylin, 31-37 hAmyin, 31-36 hAmylin, 33-36 hAmylin, 33-37 hAmylin, 28-32 sCT, (³⁰Asn ³²Tyr) 28-32 sCT, and 27-32 sCT. Herein are/is described, the C-terminal tail comprises the amino acid sequence KSNFVPTN (SEQ ID NO:62) or SNFVPTNV (SEQ ID NO:63).

It is further contemplated that no more than one, two, or three modifications such as substitutions, insertions, deletions, and/or derivatizations may be made to the C-terminal tail as described in the preceding paragraphs. The C-terminal tail of the LHC peptides may further comprise modifications or additional amino acids at the C-terminal end. Such modifications include the addition of compounds such as Lys, up to 4 Lys, L- Octylglycine, 4ABU (4-Aminobutyric acid), 9Anc (9-Amiononanoic acid), and/or groups for solubility, stability, or delivery. Examples include, but are not limited to, 33-37 hAmylin L-octylglycine, 33-37 hAmylin 4ABU, and 33-37 hAmylin 9Anc.

In a general aspect, LHC peptides comprise
(a) any of the LHC agonist loop regions as described herein;
(b) any of the LHC agonist α helix regions as described herein; and
(c) any LHC agonist C-terminal tails as described herein, with the proviso that when the loop region is from a calcitonin or calcitonin analog and the a helix region is from a calcitonin or calcitonin analog, the last position of the C-terminal tail is not Pro, Hyp, Hse or derivatives of Hse.

In another general aspect, LHC peptides comprise
(a) a loop region comprising formula (II) Xaa1 or Xaa1 with modifications at the N-terminal end;
(b) an a helix region comprising the a helix region type I or type II;
(c) a C-terminal tail represented by SEQ ID NO:61, with the proviso that when the loop region is from a calcitonin or calcitonin analog and the a helix region is from a calcitonin or calcitonin analog, the last position of the C-terminal tail is not Pro, Hyp, Hse or derivatives of Hse. The C-terminal end may comprise further modifications.

In yet another aspect, LHC peptides comprise an amino acid sequence of formula (III): Xaa1 X Xaa3 Xaa4 Xaa5 Xaa6 Y Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 Xaa23 Xaa24 Xaa25 Xaa26 Xaa27 Xaa28 Xaa29 Xaa30 Xaa31 Xaa32 (SEQ ID NO:64) wherein Xaa1 is A, C, hC, D, E, F, I, L, K, hK, R, hR, S, Hse, T, G, Q, N, M, Y, W, P,
Hyp, H, V or absent;
Xaa3 is A, D, E, N, Q, G, V, R, K, hK, hR, H, I, L, M, or absent;
Xaa4 is A, I, L, S, Hse, T, V, M, or absent;
Xaa5 is A,S,T,Hse,Y,V,I,L,or M;
Xaa6 is T, A, S, Hse, Y, V, I, L, or M;
Xaa8 is A, V, I,L, F, or M;
Xaa9 is L, T, S, Hse, V, I, or M;
Xaa10 is G, H, Q, K, R, N, hK, or hR;
Xaa11 is K, R, Q, N, M hR, or H;
Xaa12 is L, I, V, F, M, W, or Y;
Xaa 13 is A, F, Y, N, Q, S, Hse, orT;
Xaa14 is A, D, E, G, N, K,Q, R, H, hR, or hK;
Xaa15 is A, D, E, F, L, S, Y, I, V, or M;
Xaa16 is L, F, M, V, Y, or I;
Xaa17 is H, Q, N, S, Hse, T, or V;
Xaa 18 is K, hK, R, hR, H, u (Cit), or n (Orn);
Xaa19 is F, L, S, Hse,V, I, T, or absent;
Xaa20 is H, R, K, hR, hK, N, Q, or absent;
Xaa21 is T, S, Hse, V, I, L, Q, N, or absent;
Xaa22 is F, L, M, V, Y, or I;
Xaa23 is P or Hyp;
Xaa24 is P, Hyp, R,K, hR, hK, or H;
Xaa25 is T, S, Hse, V, I, L, F, or Y;
Xaa26 is N,Q, D, or E;
Xaa27 is T, V, S, F, I, or L;
Xaa28 is G or A; Xaa29 is S, Hse, T, V, I, L, or Y;
Xaa30 is E, G, K, N, D, R, hR, hK, H, or Q;
Xaa31 is A, T, S, Hse, V, I, L, F, or Y; and
Xaa32 is F, P, Y, Hse, S, T, or Hyp;
wherein X and Y are capable of creating a bond and are independently selected residues having side chains which are chemically bonded to each other to form an intramolecular linkage such as disulfide bonds; amide bond; alkyl, acids and alkyl amines which may form cyclic lactams; alkyl aldehydes or alkyl halides and alkylamines which may condensed and be reduced to form an alkyl amine or imine bridge; or side chains which may be connected to form an alkyl, alkenyl, alkynyl, ether or thioether bond. Alkyl chains may include lower alkyl groups having from about 1 to about 6 carbon atoms. Herein are/is described, the intramolecular linkage may be a disulfide, amide, imine, amine, alkyl and alkene bond. Herein are/is described, X and Y are independently selected from Ser, Asp, Glu, Lys, Orn, or Cys. Herein are/is described, X and Y are Cys and Cys. In other embodiments, X and Y are Ser and Ser. Herein are/is described, X and Y are Asp and Lys or Lys and Asp.

In yet another aspect, LHC peptides comprise an amino acid sequence of formula (IV): Xaa1 Xaa2 Xaa3 Xaa4 Xaa5 Xaa6 Xaa7 Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 P Xaa24 T N Xaa27 G S Xaa30 Xaa31 Xaa32 (SEQ ID NO:65) wherein
Xaa1 is A, C, D, F, I, K, S, T, or absent;
Xaa2 is C, D, S, or absent;
Xaa3 is A, D, N, or absent;
Xaa4 is A, L, T, or absent;
Xaa5 is A or S;
Xaa6 is T, A, S, or V;
Xaa7 is C, K, or A;
Xaa8 is A, V, L, orM;
Xaa9 is L or T;
Xaa10 is G, H, or Q;
Xaa11 is K, R, Q, or hArg;
Xaa12 is L, W, or Y;
Xaa13 is A, F, N, Q, S, or T;
Xaa14 is A, D, E, G, N, K, Q, or R;
Xaa15 is A, D, E, F, L, S, or Y;
Xaa16 is L, or F;
Xaa17 is H, Q, S, or V;
Xaa18 is K, R, hArg, u (Cit), or n (Orn);
Xaa19 is F, L, S, or absent;
Xaa20 is H, Q, or absent;
Xaa21 is T, N, or absent;
Xaa22 is F, L, M, V, or Y;
Xaa24 is P or R;
Xaa27 is T or V;
Xaa30 is E, G, K, or N;
Xaa31 is A or T; and
Xaa32 is F, P, or Y.

In yet another aspect, LHC peptides comprise an amino acid sequence of formula (V): Xaa1 Xaa2 Xaa3 Xaa4 Xaa5 T Xaa7 Xaa8 Xaa9 Xaa10 Xaa11L Xaa13 Xaa14 Xaa15 L Xaa17 Xaa18 Xaa19 Xaa20 Xaa21 Xaa22 P Xaa24 T N Xaa27 G S Xaa30 Xaa31 Xaa32, (SEQ ID NO:66) wherein
Xaa1 is A, C, F, I, K, S, or absent;
Xaa2 is C, D, or S;
Xaa3 is A, D or N;
Xaa4 is A, L or T;
Xaa5 is A or S;
Xaa7 is C or K;
Xaa8 is A or V;
Xaa9 is L or T;
Xaa10 is G, H, or Q;
Xaa11 is K, R, or hArg;
Xaa13 is A, F, N, S, or T;
Xaa14 is A, D, E, G, N, Q, or R;
Xaa15 is A, E, F, L, S, or Y;
Xaa17 is H, S, or V;
Xaa 18 is K, R, hArg, u (Cit), or n (Orn);
Xaa19 is F, L, or S;
Xaa20 is H or Q;
Xaa21 is T or N;
Xaa22 is F, L, M, V, or Y;
Xaa24 is P or R;
Xaa27 is T, or V;
Xaa30 is E, G, K, or N;
Xaa31 is A, or T; and
Xaa32 is F, P, or Y.

In a general aspect, the sequence of formula (III), (IV), or (V) further comprises 1,2,3,4,5,6,7, 8, 9,10,11,12, or more modifications of substitutions, insertions, deletions, elongations and/or derivatizations. Herein are/is described, the sequence of formula (III), (IV), or (V) comprises a Val inserted between amino acids at positions 22 and 23. Herein are/is described, the sequence of formula (III), (IV), or (V) comprises a Gln inserted between positions 22 and 23. Herein are/is described, the sequence of formula (III), (IV), or (V) comprises a sequence of Gln-Thr-Tyr between positions 22 and 23. Herein are/is described, the sequence of formula (III), (IV), or (V) comprises a sequence of Leu-Gln-Thr-Tyr (SEQ ID NO:67) between positions 22 and 23. In another general aspect, the modifications of formula (III), (IV), or (V) may be at the N-terminal end. Herein are/is described, the N-terminal portion of formula (III), (IV), or (V) has an added octylglycine. Herein are/is described, the N-terminal portion of formula (III), (IV), or (V) has an added isocap.

In yet another aspect, LHC peptides comprise an amino acid sequence of formula (VI): Xaa1 Xaa2 Xaa3 Xaa4 Xaa5 Xaa6 Xaa7 Xaa8 Xaa9 Xaa10 Xaa11 Xaa12 Xaa13 Xaa14 Xaa15 Xaa16 Xaa17 Xaa18 Xaa19 Xaa20 Xaa21 Xaa22 P Xaa24 T N Xaa27 G S Xaa30 Xaa31 Xaa32 (SEQ ID NO:68) wherein
Xaa1 is A, C, D, F, K, T, or absent;
Xaa2 is A, C, D, S, or absent;
Xaa3 is A, D, N, or absent;
Xaa4 is A, L, T, or absent;
Xaa5 is A or S;
Xaa6 is A, S, T, or V;
Xaa7 is A, C, or K;
Xaa8 is A, L, M, or V;
Xaa9 is L or T;
Xaa10 is G, H, orQ;
Xaa11 is K, Q, or R;
Xaa12 is L, W, or Y;
Xaa13 is A, N, Q, S, or T;
Xaa 14 is A, D, E, G, K, N, Q, or R;
Xaa15 is A, D, E, F, L, S, or Y;
Xaa16 is F or L;
Xaa17 is H, Q, S or V;
Xaa18 is K, or R;
Xaa19 is F, L, S, or absent;
Xaa20 is H, K, Q, or absent;
Xaa21 is Q, T, or absent;
Xaa22 is F, L, or Y;
Xaa24 is P or R;
Xaa27 is T or V;
Xaa30 is E, K or N;
Xaa31 is A or T; and
Xaa32 is F,Y, or absent.

In a general aspect, the sequence of formula (VI) further comprises 1, 2, 3, 4, 5, 6, 7, 8, 9, 10, 11, 12, or more modifications of substitutions, insertions, deletions, elongations and/or derivatizations. Herein are/is described, the sequence of formula (III), (IV), (V) or (VI) comprises a deletion at position 24.

In yet another aspect, LHC peptides comprise an amino acid sequence comprising
a) a loop region comprising Xaa1;
a) a loop region comprising the formula (II) Xaa1; wherein Xaa1 comprises an amino sequence of X Xaa2 Xaa3 Xaa4 Xaa5 Xaa6 Xaa7 Y (SEQ ID NO:72) wherein,
   Xaa2 is any amino acid or absent;
   Xaa3 is Ala, Gly, Ser, Asp or absent;
   Xaa4 is Asn, Ala, Asp, Gly or absent;
   Xaa5 is Ala, Leu, Thr, or Ser,
   Xaa6 is Ala, Ser, or Thr, and
   Xaa7 is Ala, Ser, Val, Hse, Ahb, Ahp, d-Thr, Thr, or a derivative thereof;
   X and Y are amino acids capable of creating a bond and are independently selected residues having side chains which can be chemically bonded to each other to form an intramolecular linkage such as a disulfide bond; an amide bond; a cyclic lactam formed by an alkyl acid and an alkyl amine; an alkyl amine or imine bridge formed by condensing and reducing an alkyl aldehydes or alkyl halides and alkylamines; and an alkyl, alkenyl, alkynyl, ether or thioether bond formed by connection of side chains;
b) an α helix region type II comprising the sequence X1 Xaa8 Xaa9 Xaa10 R Xaa12 Xaa13 Xaa14 Xaa15 Xaa16 Xaa17 Xaa16 Xaa19 Xaa20 Xaa21 Xaa22 P Xaa24 T N T X1 (SEQ ID NO:73) wherein
   Xaa8 is Ala or Val;
   Xaa9 is Thr, Met or Leu;
   Xaa10 is Gln, Gly, His;
   Xaa12 is Leu, or Thr;
   Xaa13 is Ala, Thr, Asn, Phe, Tyr, Ser, or Thr;
   Xaa14 is Asn, Arg, Ala, Asp, Glu, Gln, Thr, or Gly;
   Xaa15 is Phe, Leu, Ser, Glu, Ala, Asp, or Tyr,
   Xaa16 is Leu or Asp;
   Xaa17 is Val, His, Ser, Phe, or Aib;
   Xaa18 is His, Arg, Lys, Orn, hArg, Cit, hLys, Lys(for), or Lys(PEG5000);
   Xaa19 is Leu, Ser or Phe;
   Xaa20 is Gln or His;
   Xaa21 is Thr or Asn;
   Xaa22 is Tyr, Val, Phe, Leu or Met;
   Xaa24 is Arg or Pro; and
   X1 is absent or comprises 1-4 additional amino acids; and
c) a C-terminal tail comprising the sequence Xaa28 Xaa29 Xaa30 Xaa31 Xaa32 Xaa33 G Xaa35 Xaa36 Xaa37 Xaa38 (SEQ ID NO:74), wherein
   Xaa28 is Lys, Tyr, or absent;
   Xaa29 is Ser, Pro, or absent;
   Xaa30 is Ser, Pro, Arg, or absent;
   Xaa31 is Thr, or absent;
   Xaa32 is Asn, or absent;
   Xaa33 is Val, Thr, or absent;
   Xaa35 is Ser, or Glu
   Xaa36 is Asn, Lys, or Gly;
   Xaa37 is Thr, Phe, or Ala;
   Xaa38 is Tyr, Phe, Pro, or absent

In general, amylin agonists or amylin agonist analogs are recognized as referring to compounds which, by directly or indirectly interacting or binding with one or more receptors, mimics an action of amylin. Accordingly, compounds herein are/is described act as an agonist for at least one biological effect of amylin, or bind to at least one of the receptors of amylin. Conversely, amylin antagonists by directly or indirectly interacting or binding with one or more receptors, suppresses an action of amylin. Such interactions or binding events include those that affect ghrelin levels.

Amylin antagonists include AC66 (sCT[8-32]) (SEQ ID NO:172) and derivatives such as AC187 (Ac 30Asn, 32Tyr-sCT[8-32]) (SEQ ID NO: 173) a 25 amino acid peptide fragment of salmon calcitonin, developed as a selective amylin receptor antagonist over CGRP receptors. Other antagonists include antagonists described in U.S. Patent Nos. 5,625,032 and 5,580,953. Such antagonist compounds include those comprising formula (VII): X-R1-Thr-Gln-R2-Leu-Ala-Asn-R3-Leu-Val-Arg-Leu-Gln-Thr-Tyr-Pro-Arg-Thr-Asn-Val-Gly-R4-Asn-Thr-Tyr-NH2 (SEQ ID NO: 174)
wherein
R1 is Ala or a bond;
R2 is Arg, Gln, Lys, Asn or Leu;
R3 is Gln, Glu, Asn, Asp or Phe;
R4 is Ala or Ser, and
X is hydrogen or an acetyl group.

Amylin antagonists may be acetylated or non-acetylated at the N-terminus and include acid as well as amide forms of the molecule. Examples of amylin antagonists include, but are not limited to, acetyl-Ala Thr Gln Arg Leu Ala Asn Glu Leu Val Arg Leu Gln Thr Tyr Pro Arg Thr Asn Val Gly Ser Asn Thr Tyr (SEQ ID NO: 15), Ala Thr GlnGln Leu Ala Asn Gln Leu Val Arg Leu Gln Thr Tyr Pro Arg Thr Asn Val Gly Ser Asn Thr Tyr (SEQ ID NO: 176), Ala Thr Gln Leu Leu Ala Asn Gln Leu Val Arg Leu Gln Thr Tyr Pro Arg Thr Asn Val Gly Ser Asn Thr Tyr (SEQ ID NO: 177), Ala Thr Gln Arg Leu Ala Asn Gln Leu Val Arg Leu Gln Thr Tyr Pro Arg Thr Asn Val Gly Ser Asn Thr Tyr (SEQ ID NO: 178), Ala Thr Gin Leu Leu Ala Asn Glu Leu Val Arg Leu Gln Thr Tyr Pro Arg Thr Asn Val Gly Ser Asn Thr Tyr (SEQ ID NO: 179), Ala Thr Gln Gln Leu Ala Asn Glu Leu Val Arg Leu Gln Thr Tyr Pro Arg Thr Asn Val Gly Ser Asn Thr Tyr (SEQ ID NO: 180).

Methods of testing compounds for amylin activity are known in the art. Exemplary screening methods and assays for testing amylin agonists or antagonists are described in the Examples, particularly Example 4 herein, and in U.S. Patent Nos. 5,264,372 and 5,686,411.

Activity as amylin agonists and/or analogs can be confirmed and quantified by performing various screening assays, including the nucleus accumbens receptor binding assay, followed by the soleus muscle assay, a gastric emptying assay, or by the ability to induce hypocalcemia or reduce postprandial hyperglycemia in mammals.

The receptor binding assay, a competition assay that measures the ability of compounds to bind specifically to membrane-bound amylin receptors, is described in U.S. Pat. Nos. 5,264,372 and 5,686,411. A preferred source of the membrane preparations used in the assay is the basal forebrain which comprises membranes from the nucleus accumbens and surrounding regions. Compounds being assayed compete for binding to these receptor preparations with 125I Bolton Hunter rat amylin. Competition curves, wherein the amount bound (B) is plotted as a function of the log of the concentration of ligand, are analyzed by computer using analyses by nonlinear regression to a 4-parameter logistic equation (INPLOT program; GraphPad Software, San Diego, Calif.) or the ALLFIT program of DeLean et al. (ALLFIT, Version 2.7 (NIH, Bethesda, Md. 20892)). Munson and Rodbard (1980) Anal. Biochem. 107:220-239.

Assays of biological activity of amylin agonists/analogs in the soleus muscle may be performed using previously described methods (Leighton et al. (1988) Nature 335:632-635; Cooper et al. (1988) Proc. Natl. Acad Sci. USA 85:7763-7766), in which amylin agonist activity may be assessed by measuring the inhibition of insulin-stimulated glycogen synthesis. In brief, an exemplary method includes soleus muscle strips prepared from 12-h fasted male Wistar rats. The tendons of the muscles are ligated before attachment to stainless steel clips. Muscle strips are pre-incubated in Erlenmeyer flasks containing 3.5 ml Krebs-Ringer bicarbonate buffer, 7mM N-2-hydroxyethyl-peperazine-N'-2-ethane-sulphonic acid, pH 7.4, and 5.5 mM pyruvate. Flasks are sealed and gassed continuously with O₂ and CO₂ in the ratio 19:1 (v/v). After pre-incubation of muscles in this medium for 30 min at 37°C in an oscillating water bath, the muscles strips are transferred to similar vials containing identical medium (except pyruvate) with added [U-14C] glucose (0.5 µCi/ml) and insulin (100 µU/ml). The flasks are sealed and re-gassed for an initial 15 min in a 1-h incubation. At the end of the incubation period, muscles are blotted and rapidly frozen in liquid N2. The concentration of lactate in the incubation medium can be determined spectrophotometrically and [U-14C]glucose incorporation in glycogen measured. Amylin antagonist activity is assessed by measuring the resumption of insulin-stimulated glycogen synthesis in the presence of 100 nM rat amylin and an amylin antagonist.

Methods of measuring the rate of gastric emptying are disclosed in, for example, Young et al. In a phenol red method, conscious rats receive by gavage an acoloric gel containing methyl cellulose and a phenol red indicator. Twenty minutes after gavage, animals are anesthetized using halothane, the stomach exposed and clamped at the pyloric and lower esophageal sphincters, removed and opened into an alkaline solution. Stomach content may be derived from the intensity of the phenol red in the alkaline solution, measured by absorbance at a wavelength of 560 nm. In a tritiated glucose method, conscious rats are gavaged with tritiated glucose in water. The rats are gently restrained by the tail, the tip of which is anesthetized using lidocaine. Tritium in the plasma separated from tail blood is collected at various timepoints and detected in a beta counter. Test compounds are normally administered about one minute before gavage.

Amylin agonist and antagonist compounds may exhibit activity in the receptor binding assay on the order of less than about 1 to 5 nM, preferably less than about 1 nM and more preferably less than about 50 pM. In the soleus muscle assay, amylin agonist compounds may show EC50 values on the order of less than about 1 to 10 micromolar. In the soleus muscle assay, amylin antagonists may show IC₅₀ values on the order of less than about 1 to 2 micromolar. In the gastric emptying assays, preferred agonist compounds show ED₅₀ values on the order of less than 100 µg/rat. Antagonist compounds would show no effect or the opposite effect in the gastric emptying assay.

In one exemplary method of making the compounds, compounds herein are/is described may be prepared using standard solid-phase peptide synthesis techniques and preferably an automated or semiautomated peptide synthesizer. Typically, using such techniques, an α-N-carbamoyl protected amino acid and an amino acid attached to the growing peptide chain on a resin are coupled at room temperature in an inert solvent such as dimethylformamide, N-methylpyrrolidinone or methylene chloride in the presence of coupling agents such as dicyclohexylcarbodiimide and 1-hydroxybenzotriazole in the presence of a base such as diisopropylethylamine. The α-N-carbamoyl protecting group is removed from the resulting peptide-resin using a reagent such as trifluoroacetic acid or piperidine, and the coupling reaction repeated with the next desired N-protected amino acid to be added to the peptide chain. Suitable N-protecting groups are well known in the art, with t-butyloxycarbonyl (tBoc) and fluorenylmethoxycarbonyl (Fmoc) being preferred herein. Other methods of synthesizing or expressing amylin and amylin agonists and purifying them are known to the skilled artisan.

### Dosage/Formulation

Anti-obesity agents and weight-inducing agents (herein referred to in this section as the "compounds") may be administered alone or in combination with pharmaceutically acceptable carriers or excipients, in either single or multiple doses. These pharmaceutical compounds may be formulated with pharmaceutically acceptable carriers or diluents as well as any other known adjuvants and excipients in accordance with conventional techniques such as those disclosed in Remington's Pharmaceutical Sciences by E. W. Martin. See also Wang et al. (1988) J. of Parenteral Sci. and Tech., Technical Report No. 10, Supp. 42:2S.

In general, the compounds may be formulated into a stable, safe pharmaceutical composition for administration to a patient. Pharmaceutical formulations contemplated for use in the invention may comprise approximately 0.01 to 1.0% (w/v), in certain cases 0.05 to 1.0%, of the compound, approximately 0.02 to 0.5% (w/v) of an acetate, phosphate, citrate or glutamate buffer allowing a pH of the final composition of from about 3.0 to about 7.0; approximately 1.0 to 10% (w/v) of a carbohydrate or polyhydric alcohol tonicifier and, optionally, approximately 0.005 to 1.0% (w/v) of a preservative selected from the group consisting of m-cresol, benzyl alcohol, methyl, ethyl, propyl and butyl parabens and phenol. Such a preservative is generally included if the formulated peptide is to be included in a multiple use product.

Herein are/is described, a pharmaceutical formulation may contain a range of concentrations of the compound, e.g., between about 0.01% to about 98% w/w, or between about 1 to about 98% w/w, or preferably between 80% and 90% w/w, or preferably between about 0.01% to about 50% w/w, or more preferably between about 10% to about 25% w/w. A sufficient amount of water for injection may be used to obtain the desired concentration of solution.

Additional tonicifying agents such as sodium chloride, as well as other known excipients, may also be present, if desired. In some cases, such excipients are useful in maintenance of the overall tonicity of the compound. An excipient may be included in the presently described formulations at various concentrations. For example, an excipient may be included in the concentration range from about 0.02% to about 20% w/w, preferably between about 0.02% and 0.5% w/w, about 0.02% to about 10% w/w, or about 1 % to about 20% w/w. In addition, similar to the present formulations themselves, an excipient may be included in solid (including powdered), liquid, semi-solid or gel form.

The pharmaceutical formulations may be composed in various forms, e.g., solid, liquid, semisolid or liquid. The term "solid", as used herein, is meant to encompass all normal uses of this term including, for example, powders and lyophilized formulations. The presently described formulations may be lyophilized.

The terms buffer, buffer solution and buffered solution, when used with reference to hydrogen-ion concentration or pH, refer to the ability of a system, particularly an aqueous solution, to resist a change of pH on adding acid or alkali, or on dilution with a solvent Characteristic of buffered solutions, which undergo small changes of pH on addition of acid or base, is the presence either of a weak acid and a salt of the weak acid, or a weak base and a salt of the weak base. An example of the former system is acetic acid and sodium acetate. The change of pH is slight as long as the amount of hydronium or hydroxyl ion added does not exceed the capacity of the buffer system to neutralize it

As described herein, a variety of liquid vehicles are suitable for use in the formulations of peptidic anti-obesity agents, for example, water or an aqueous/organic solvent mixture or suspension.

The stability of a peptide formulation for use in the present invention is enhanced by maintaining the pH of the formulation in the range of about 3.0 to about 7.0 when in liquid form. In certain embodiments, the pH of the formulation is maintained in the range of about 3.5 to 5.0, or about 3.5 to 6.5, in some embodiments from about 3.7 to 4.3, or about 3.8 to 4.2. In some embodiments, pH may be about 4.0. While not seeking to be bound by this theory, it is presently understood that where the pH of the pharmaceutical formulation exceeds 5.5, chemical degradation of the peptide may be accelerated such that the shelf life is less than about two years.

In certain embodiments, the buffer with the anti-obesity agents is an acetate buffer (preferably at a final formulation concentration of from about 1-5 to about 60 mM), phosphate buffer (preferably at a final formulation concentration of from about 1-5 to about to about 30 mM) or glutamate buffer (preferably at a final formulation concentration of from about 1-5 to about to about 60 mM). In some embodiments, the buffer is acetate (preferably at a final formulation concentration of from about 5 to about 30 mM).

A stabilizer may be included in the formulations of anti-obesity agents but, and importantly, is not necessarily needed. If included, however, a stabilizer useful in the practice of the present invention is a carbohydrate or a polyhydric alcohol. A suitable stabilizer useful in the practice of the present invention is approximately 1.0 to 10% (w/v) of a carbohydrate or polyhydric alcohol. The polyhydric alcohols and carbohydrates share the same feature in their backbones, i.e., -CHOH-CHOH-, which is responsible for stabilizing the proteins. The polyhydric alcohols include such compounds as sorbitol, mannitol, glycerol, and polyethylene glycols (PEGs). These compounds are straight-chain molecules. The carbohydrates, such as mannose, ribose, sucrose, fructose, trehalose, maltose, inositol, and lactose, on the other hand, are cyclic molecules that may contain a keto or aldehyde group. These two classes of compounds have been demonstrated to be effective in stabilizing protein against denaturation caused by elevated temperature and by freeze-thaw or freeze-drying processes. Suitable carbohydrates include: galactose, arabinose, lactose or any other carbohydrate which does not have an adverse affect on a diabetic patient, i.e., the carbohydrate is not metabolized to form unacceptably large concentrations of glucose in the blood. Such carbohydrates are well known in the art as suitable for diabetics. Sucrose and fructose are suitable for use with the compound in non-diabetic applications (e.g. treating obesity).

In certain embodiments of the use, if a stabilizer is included, the compound is stabilized with a polyhydric alcohol such as sorbitol, mannitol, inositol, glycerol, xylitol, and polypropylene/ethylene glycol copolymer, as well as various polyethylene glycols (PEG) of molecular weight 200, 400, 1450, 3350, 4000, 6000, and 8000). Mannitol is the preferred polyhydric alcohol in some embodiments. Another useful feature of the lyophilized formulations herein are/is described is the maintenance of the tonicity of the lyophilized formulations described herein with the same formulation component that serves to maintain their stability. In some embodiments, mannitol is the preferred polyhydric alcohol used for this purpose.

The United States Pharmacopeia (USP) states that anti-microbial agents in bacteriostatic or fungistatic concentrations must be added to preparations contained in multiple dose containers. They must be present in adequate concentration at the time of use to prevent the multiplication of microorganisms inadvertently introduced into the preparation while withdrawing a portion of the contents with a hypodermic needle and syringe, or using other invasive means for delivery, such as pen injectors. Antimicrobial agents should be evaluated to ensure compatibility with all other components of the formula, and their activity should be evaluated in the total formula to ensure that a particular agent that is effective in one formulation is not ineffective in another. It is not uncommon to find that a particular antimicrobial agent will be effective in one formulation but not effective in another formulation.

A preservative is, in the common pharmaceutical sense, a substance that prevents or inhibits microbial growth and may be added to pharmaceutical formulations for this purpose to avoid consequent spoilage of the formulation by microorganisms. While the amount of the preservative is not great, it may nevertheless affect the overall stability of the peptide.

While the preservative for use in the pharmaceutical compositions can range from 0.005 to 1.0% (w/v), in some embodiments range for each preservative, alone or in combination with others, is: benzyl alcohol (0.1-1.0%), orm-cresol (0.1-0.6%), or phenol (0.1-0.8%) or combination of methyl (0.05-0.25%) and ethyl or propyl or butyl (0.005%-0.03%) parabens. The parabens are lower alkyl esters of para-hydroxybenzoic acid. A detailed description of each preservative is set forth in Remington's Pharmaceutical Sciences by Martin.

Pramlintide, human ^{25,28,29}Pro-amylin, does not have a tendency to adsorb onto the glass in a glass container when in a liquid form, therefore, a surfactant is not required to further stabilize the pharmaceutical formulation. However, with regard to compounds which do have such a tendency when in liquid form, a surfactant should be used in their formulation. These formulations may then be lyophilized. Surfactants frequently cause denaturation of protein, both of hydrophobic disruption and by salt bridge separation. Relatively low concentrations of surfactant may exert a potent denaturing activity, because of the strong interactions between surfactant moieties and the reactive sites on proteins. However, judicious use of this interaction can stabilize proteins against interfacial or surface denaturation. Surfactants which could further stabilize the peptide may optionally be present in the range of about 0.001 to 0.3% (w/v) of the total formulation and include polysorbate 80 (i.e., polyoxyethylene(20) sorbitan monooleate), CHAPS® (i.e., 3-[(3-cholamidopropyl) dimethylammonio] 1-propanesulfonate), Brij® (e.g., Brij 35, which is (polyoxyethylene (23) lauryl ether), poloxamer, or another non-ionic surfactant.

It may also be desirable to add sodium chloride or other salt to adjust the tonicity of the pharmaceutical formulation, depending on the tonicifier selected. However, this is optional and depends on the particular formulation selected. Parenteral formulations preferably may be isotonic or substantially isotonic.

A preferred vehicle for parenteral products is water. Water of suitable quality for parenteral administration can be prepared either by distillation or by reverse osmosis. Water for injection is the preferred aqueous vehicle for use in the pharmaceutical formulations.

It is possible that other ingredients may be present in the pharmaceutical formulations. Such additional ingredients may include, e.g., wetting agents, emulsifiers, oils, antioxidants, bulking agents, tonicity modifiers, chelating agents, metal ions, oleaginous vehicles, proteins (e.g., human serum albumin, gelatin or proteins) and a zwitterion (e.g., an amino acid such as betaine, taurine, arginine, glycine, lysine and histidine). Additionally, polymer solutions, or mixtures with polymers provide the opportunity for controlled release of the peptide. Such additional ingredients, of course, should not adversely affect the overall stability of the pharmaceutical formulation herein disclosed.

Containers are also an integral part of the formulation of an injection and may be considered a component, for there is no container that is totally inert, or does not in some way affect the liquid it contains, particularly if the liquid is aqueous. Therefore, the selection of a container for a particular injection must be based on a consideration of the composition of the container, as well as of the solution, and the treatment to which it will be subjected. Adsorption of the peptide to the glass surface of the vial can also be minimized, if necessary, by use of borosilicate glass, for example, Wheaton Type I borosilicate glass #33 (Wheaton Type I-33) or its equivalent (Wheaton Glass Co.). Other vendors of similar borosilicate glass vials and cartridges acceptable for manufacture include Kimbel Glass Co., West Co., Bünder Glas GMBH and Forma Vitrum. The biological and chemical properties of the compound may be stabilized by formulation and lyophilization in a Wheaton Type I-33 borosilicate serum vial to a final concentration of 0.1 mg/ml and 10 mg/ml of the compound in the presence of 5% mannitol, and 0.02% Tween 80.

For formulations to be delivered by injection, in order to permit introduction of a needle from a hypodermic syringe into a multiple-dose vial and provide for resealing as soon as the needle is withdrawn, the open end of each vial is preferably sealed with a rubber stopper closure held in place by an aluminum band.

Stoppers for glass vials, such as, West 4416/50, 4416/50 (Teflon faced) and 4406/40, Abbott 5139 or any equivalent stopper can be used as the closure for pharmaceutical for injection. For formulations comprising peptidic anti-obesity agents, these stoppers are compatible with the peptide as well as the other components of the formulation. The inventors have also discovered that these stoppers pass the stopper integrity test when tested using patient use patterns, e.g., the stopper can withstand at least about 100 injections. Alternatively, the peptide can be lyophilized in to vials, syringes or cartridges for subsequent reconstitution. Liquid formulations described herein can be filled into one or two chambered cartridges, or one or two chamber syringes.

Each of the components of the pharmaceutical formulation described above is known in the art and is described in Pharmaceutical Dosage Forms: Parenteral Medications, Vol. 1, 2nd ed., Avis et al. Ed., Mercel Dekker, New York, N.Y. 1992.

The manufacturing process for the above liquid formulations generally involves compounding, sterile filtration and filling steps. The compounding procedure involves dissolution of ingredients in a specific order (preservative followed by stabilizer/tonicity agents, buffers and peptide) or dissolving at the same time.

Alternative formulations, e.g., non-parenteral, may not require sterilization. However, if sterilization is desired or necessary, any suitable sterilization process can be used in developing the peptide pharmaceutical formulation described herein. Typical sterilization processes include filtration, steam (moist heat), dry heat, gases (e.g., ethylene oxide, formaldehyde, chlorine dioxide, propylene oxide, beta-propiolacctone, ozone, chloropicrin, peracetic acid methyl bromide and the like), exposure to a radiation source, and aseptic handling. Filtration is the preferred method of sterilization for described herein. The sterile filtration involves filtration through 0.45 µm and 0.22 µm (1 or 2) which may be connected in series. After filtration, the solution is filled into appropriate vials or containers.

In one embodiment, the anti-obesity agents are to be administered peripherally to the subjects. In some embodiments, the liquid pharmaceutical formulations used in the present invention are intended for parenteral administration. Suitable routes of administration include intramuscular, intravenous, subcutaneous, intradermal, intraarticular, intrathecal and the like. In some embodiments, the subcutaneous route of administration is preferred. In certain embodiments, mucosal delivery is also preferred. These routes include, but are not limited to, oral, nasal, sublingual, pulmonary and buccal routes which may include administration of the peptide in liquid, semi-solid or solid form. For formulations comprising peptidic anti-obesity agents, administration via these routes requires substantially more peptide to obtain the desired biological effects due to decreased bioavailability compared to parenteral delivery. In addition, parenteral controlled release delivery can be achieved by forming polymeric microcapsules, matrices, solutions, implants and devices and administering them parenterally or by surgical means. Examples of controlled release formulations are described in U.S. Patent Nos. 6,368,630, 6,379,704, and 5,766,627. These dosage forms may have a lower bioavailability due to entrapment of some of the peptide in the polymer matrix or device. See e.g., U.S. Pat. Nos. 6,379,704, 6,379,703, and 6,296,842.

The compounds may be provided in dosage unit form containing an amount of the compound with or without insulin or glucose (or a source of glucose) that will be effective in one or multiple doses to control the effects of ghrelin. Therapeutically effective amounts of the compounds for the treatment of ghrelin-associated diseases or disorders are those sufficient to treat, prevent, or ameliorate the physiological effects of undesirable levels of ghrelin.

As will be recognized by those in the field, an effective amount of the anti-obesity agents will vary with many factors including the age and weight of the patient, the patient's physical condition, the condition to be treated, and other factors. An effective amount of the anti-obesity agents will also vary with the particular combination administered. As described herein, administration of the agents in combination may allow for a reduced amount of any of the administered agents to be an effective amount.

However, typical doses may contain from a lower limit of about 1 µg, 5 µg, 10 µg, 50 µg to 100 µg to an upper limit of about 100 µg, 500 µg, 1 mg, 5 mg, 10 mg, 50 mg or 100 mg of the pharmaceutical compound per day. Also contemplated are other dose ranges such as 0.1 µg to 1 mg of the compound per dose. The doses per day may be delivered in discrete unit doses, provided continuously in a 24 hour period or any portion of that the 24 hours. The number of doses per day may be from 1 to about 4 per day, although it could be more. Continuous delivery can be in the form of continuous infusions. Exemplary doses and infusion rates include from 0.005 nmol/kg to about 20 nmol/kg per discrete dose or from about 0.01/pmol/kg/min to about 10 pmol/kg/min in a continuous infusion. These doses and infusions can be delivered by intravenous administration (i.v.) or subcutaneous administration (s.c.). Exemplary total dose/delivery of the pharmaceutical composition given i.v. may be about 2 µg to about 8 mg per day, whereas total dose/delivery of the pharmaceutical composition given s.c may be about 6 µg to about 6 mg per day.

Leptin and leptin derivatives may be administered, for example, at a daily dosage of from about 0.01 mg/kg to about 20 mg/kg, in some cases, from about 0.01 mg/kg to about 0.3 mg/kg. Administration may be by injection of a single dose or in divided doses.

Rimonabant may be administered, for example, at a daily dosage of from about 0.01 mg/kg to about 8 mg/kg, in some instances from about 0.3 mg/kg to about 3 mg/kg of body weight in a single dose or in divided doses 2 to 3 times per day, or in sustained release form.

The following Examples are provided to illustrate, but not limit, the invention.

### EXAMPLES

### EXAMPLE 1

Diet-induced obesity (DIO) in the in the Sprague-Dawley rat is a valuable model for the study of obesity and regulation of energy homeostasis. These rats were developed from a line of (Crl:CD®(SD)BR) rats that are prone to become obese on a diet relatively high in fat and energy. See, for example, Levin (1994) Am. J. Physiol. 267:R527-R535, Levin et al. (1997) Am. J. Physiol. 273:R725-R730. DIO male rats were obtained from Charles River Laboratories, Inc. (Wilmington, MA). The rats were housed individually in shoebox cages at 22 °C in a 12/12-hour light dark cycle. Rats were maintained *ad-libitum* on a moderately high fat diet (32% kcal from fat; Research Diets D1226B) for 6-7 weeks prior to drug treatment. At the end of the fattening period (prior to drug administration), the animals typically achieve a mean body weight of about 500 g.

The DIO animals were divided into four treatment groups. Each group was implanted with subcutaneous osmotic mini-pumps (DURECT Corp., Cupertino, CA) designed to deliver vehicle, leptin (500 µg/kg/day), amylin (100 µg/kg/day) or leptin (500 µg/kg/day) + amylin (100 µg/kg/day) for a 14 day period. As described herein, amylin acts on structures in the hindbrain involved in food intake and/or body weight modulation and leptin acts on structures in the hypothalamus involved in food intake and/or body weight modulation. Food intake and body weight were recorded daily. Body composition was measured prior to and after drug treatment using NMR echo Medical Systems, Houston, TX). Indirect calorimetry was used to measure changes in energy expenditure on days 4,5 and 6 of drug treatment (Oxymax; Columbus Instruments, Columbus, OH). All data are represented as mean ± SEM. Analysis of variance (ANOVA) and post-hoc tests were used to test for group difference. A *P*-value <0.05 was considered significant. Statistical analysis and graphing were performed using PRISM® 4 for Windows (GraphPad Software, Inc., San Diego, CA). In some early studies, SYSTAT® for Windows (Systat Soflware, Inc., Point Richmond CA) was used for analysis.

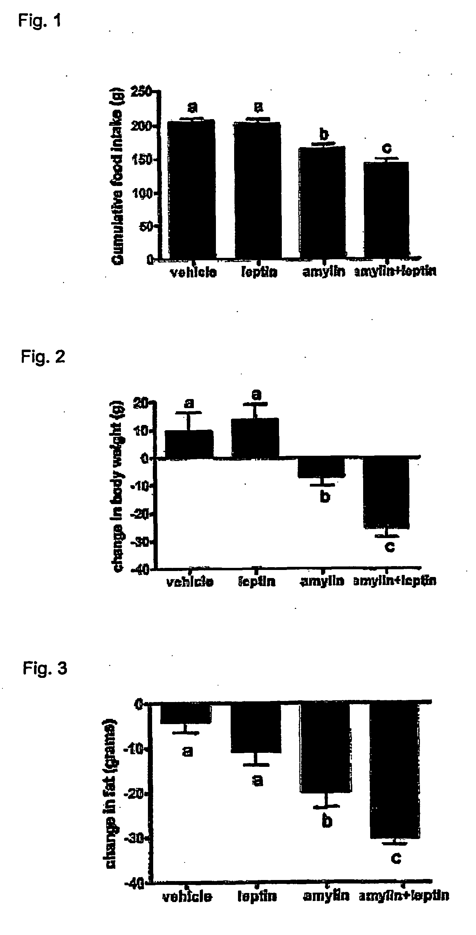

Figures 1 and 2 show the effects of amylin and leptin on cumulative food intake and total changes in body weight after 14 days of drug administration. Of particular interest is that (1) leptin, the forebrain acting agent, was ineffective on its own in this obesity model (no effect on either food intake or body weight), and (2) rats treated with the combination of amylin and leptin consumed significantly less food and lost significantly more weight relative to rats treated with either vehicle or amylin or leptin alone (p<0.05; different letters indicate that groups differed significantly from one another).

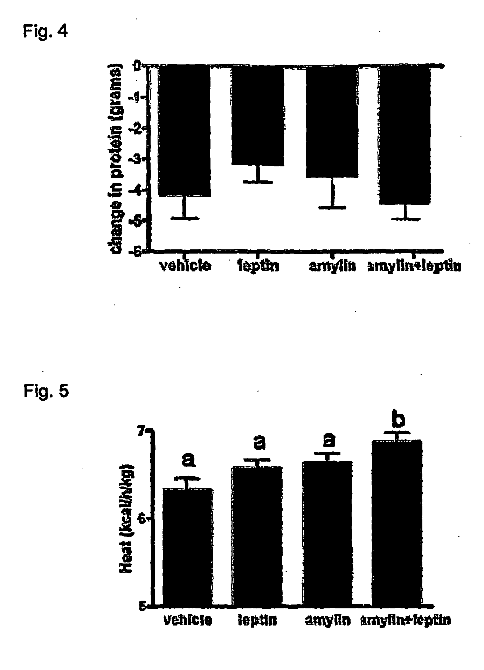

Similar effects were observed on body composition. Figure 3 depicts changes in body fat produced by the treatments and Figure 4 depicts changes in body protein produced by the treatments. Fat loss in animals treated with the combination of amylin+leptin was significantly greater than that in animals treated with either individual agent or vehicle (p<0.05). These changes in body fat were not accompanied by significant decreases in lean tissue (p>0.05; Figure 4).

Figure 5 depicts changes in energy expenditure during the dark cycle of the treatment groups. While drug (or diet)-induced reduction in food intake and body weight is often accompanied by a slowing of metabolic rate (Heat, kcal/h/kg), rats treated with the combination of leptin and amylin had a significantly higher metabolic rate during the dark cycle relative to the other groups (p<0.05). Thus, simultaneously targeting hindbrain and forebrain feeding centers with the amylin + leptin combination resulted in a significant and sustained reduction in food intake, body weight and body fat while increasing metabolic rate. In addition, the reductions in body weight and body fat were not accompanied by a reduction in lean tissue mass.

### EXAMPLE 2

Another series of experiments were performed to further explore the synergistic effects of the combination of amylin and leptin on changes in body weight and body composition. In-bred DIO (Levin) rats were obtained from Charles Rivers Labs for these studies. These rats were developed by Barry Levin from a line of Crl:CD®(SD)BR rats that are prone to become obese on a diet relatively high in fat and energy. They were housed individually in shoebox cages at 22 C in a 12/12-hour light dark cycle. Rats were maintained *ad-libitum* on a moderately high fat diet(32% kcal from fat; Research Diets D1226B) for approximately 6 weeks prior to drug treatment and throughout the experiment with the exception of pair-fed controls (PF). PF rats were restricted to the intake of either the amylin-treated group. Prior to drug administration rats had typically attained a mean body weight of 500 g.

The animals were divided into treatment groups counter-balanced for body weight and were implanted with subcutaneous osmotic mini-pumps (Durect Corp., Cupertino, CA). Each rat was implanted with two mini-pumps containing drug or the appropriate vehicle. Rat amylin (AC0128; Lot#28) was dissolved in 50% DMSO in sterile water and murine leptin (Peprotech, catalog#450-31) was dissolved in sterile water. The pumps were designed to deliver vehicle, amylin at 100 µg/kg/day or murine leptin at 500 µg/kg/day for a 14 day period.

Each group was implanted with subcutaneous osmotic mini-pumps (DURECT Corp., Cupertino, CA) designed to deliver vehicle, leptin (500 µg/kg/day), amylin (100 µg/kg/day) or leptin (500 µg/kg/day) + amylin (100 µg/kg/day) for a 14 day period. Body weight and food intake were recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment-grams of body fat at baseline = change in grams of body fat) and changes in % body composition for fat and dry lean tissue (e.g., % body fat after treatment -% body fat at baseline = change in % body fat).

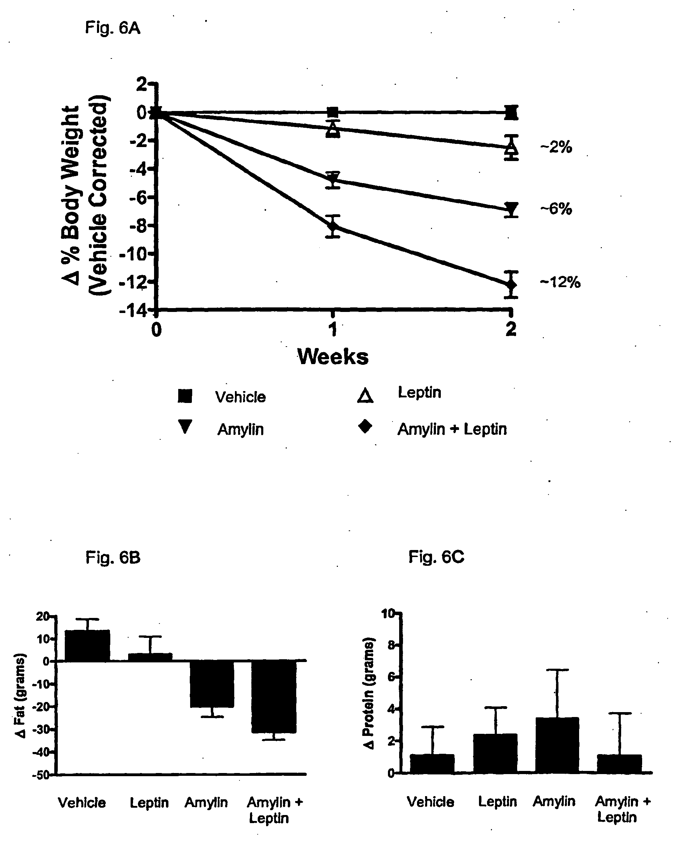

The graph in Figure 6A depicts vehicle-corrected changes in percent body weight of the treatment groups over the two weeks of treatment. In this experiment, leptin administration resulted in an overall 2% decrease in body weight and amylin administration resulted in an overall 6% decease in body weight. Notably, the percent decrease in body weight in response to administration of a combination of amylin and leptin was about 12%, an effect greater than the combined effect from the individual agents administered alone. Accordingly, amylin and leptin acted synergistically to reduce body weight. Figures 6B and 6C depict changes in body fat and changes in body protein, respectively, produced after the two weeks of treatment. Again, the reduction in body fat as a result of the combination of agents is more than the combined reduction in body fat as a result of the individual agents. These changes in body fat were not accompanied decreases in body protein but rather by a gain in percent protein. These results support a metabolic effect of the combination of agents as well as a weight reducing effect.

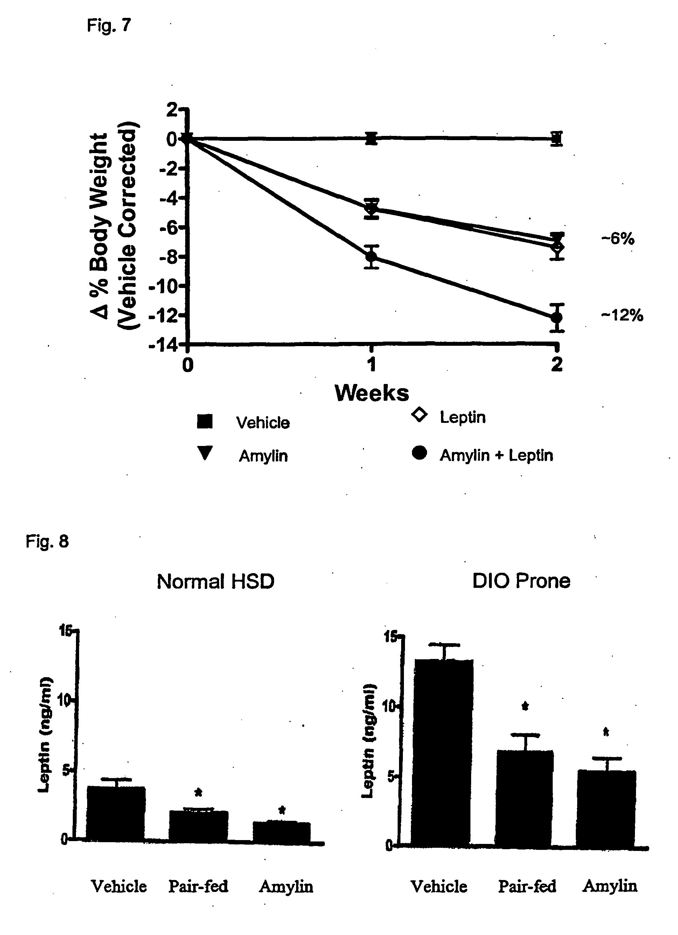

Amylin is known to have an anorectic effect on a recipient In order to examine the effect of amylin + leptin in the context of an anorectic effect of amylin, a pair-feeding experiment was performed. DIO rats and drug treatment groups were established as described above. DIO rats in the vehicle, amylin, and amylin + leptin treatment groups had *ad libitum* access to food, while intake in the pair-fed leptin treatment was restricted to the amount consumed by the amylin-treated group. Body weight was recorded daily for the two weeks of treatment. As shown in Figure 7, the amylin treatment group and the pair-fed, leptin treatment group both had approximately a 6% decrease in body weight relative to vehicle control. This result is consistent with the previous result of leptin having little or no effect on body weight in the DIO animals. The combination of amylin + leptin results in a decrease of about 12 % in body weight relative to vehicle control. Accordingly, the pair-fed experiment demonstrates that the combination of amylin + leptin reduces body weight over and above that conferred by caloric restriction.

### EXAMPLE 3 (not within the scope of the invention)

As discussed herein, serum leptin levels in the majority of humans with obesity are high, and a state of leptin resistance is thought to exist in these individuals. Plasma leptin levels and leptin resistance were examined in normal Harlan Sprague-Dawley (HSD) and in DIO prone rats.

DIO prone and normal HSD rats were divided into three treatment groups. Two groups were implanted with subcutaneous osmotic mini-pumps (DURECT Corp., Cupertino, CA) designed to deliver vehicle or amylin (100 µg/kg/day) and the third group was pair-fed to the amount consumed by the amylin-treated group for a 14 day period. Serum leptin levels were determined by immunoassays using a commercial kit (Linco Research, Inc., St. Charles, MO). As shown in Figure 8, the serum leptin level in the DIO prone animals is approximately three-times higher than that in the normal HSD animals. Thus, DIO prone rats are hyper-leptinemic. Both amylin treatment and caloric restriction to the amount of food eaten by the amylin-treated animals significantly reduced plasma leptin levels in both the DIO prone and the normal animals.

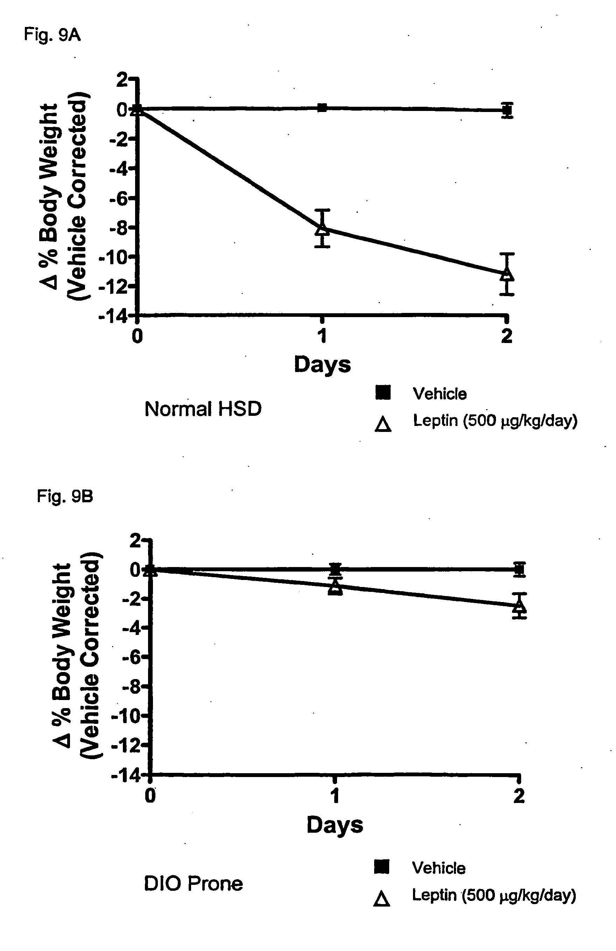

Normal, lean HSD rats were divided into two treatment groups. Each group was implanted with subcutaneous osmotic mini-pumps (DURECT Corp., Cupertino, CA) designed to deliver either vehicle or leptin (500 µg/kg/day) for a 14 day period and body weight was recorded weekly. As shown in Figure 9, the ineffective dose of leptin (500 mg/kg/day) in DIO prone animals, elicited a significant and sustained reduction in body weight in normal HSD rats. The DIO prone animals described herein appear resistant to the weight reducing effect of leptin.

### Comparative EXAMPLE 4

To demonstrate effects of the combination of amylin and a serotonergic/noradrenergic reuptake inhibitor on changes in body weight and body composition, DIO male rats were fattened and divided into four treatment groups, as described in Example 2. Rat amylin was dissolved in 50% DMSO in sterile water and sibutramine was dissolved in sterile water. Each group was implanted with subcutaneous osmotic mini-pumps designed to deliver vehicle, sibutramine (3 mg/kg/day) or amylin (100 µg/kg/day) for a 14 day period. Body weight and food intake were recorded daily. Body composition was measured prior to and after drug treatment using NMR(Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment -% body fat at baseline = change in % body fat).

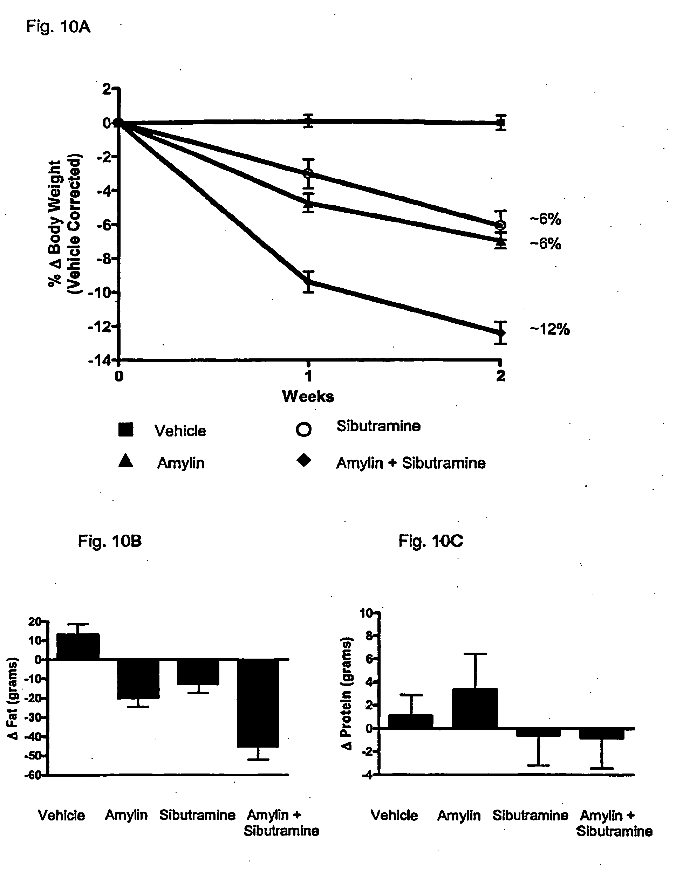

The graph in Figure 10A depicts vehicle-corrected changes in percent body weight of the treatment groups over the two weeks of treatment. Sibutramine administration alone and amylin administration alone resulted in about a 6% decease in body weight The percent decrease in body weight in response to administration of a combination of amylin and sibutramine was about 12%. Figures 10B and 10C depict changes in body fat and changes in body protein, respectively, produced after the two weeks of treatment Fat mass loss was evident with the treatment of either amylin alone or sibutramine alone, and a synergistic effect was obtained when both amylin and sibutramine were administered in combination (Fig. 10B). Administration of amylin alone resulted in an increase in lean (protein) mass. Lean (protein) mass was relatively unchanged when sibutramine was administered alone or in combination with amylin (Fig. 10C). These results support a metabolic effect of the combination of agents as well as a weight reducing effect.

The combination of amylin and catecholaminergic agonist, phentermine, were also tested for effects on changes in body weight and body composition. Phentermine is classically referred to as a catecholaminergic agonist as it actually hits NA/5-HT receptors. DIO male rats were fattened and divided into four treatment groups, as described above. Each group was implanted with subcutaneous osmotic mini-pumps and/or inserted with an oral gavage, designed to deliver vehicle, phentermine (10 mg/kg/day), amylin (100 µg/kg/day) or phentermine (10 mg/kg/day) + amylin (100 µg/kg/day) for a 14 day period. The mini-pump contained either vehicle (50% DMSO in water) or amylin while the oral gavage administered either sterile water or phentermine. Body weight was recorded daily and body composition was measured prior to and after drug treatment using NMR.

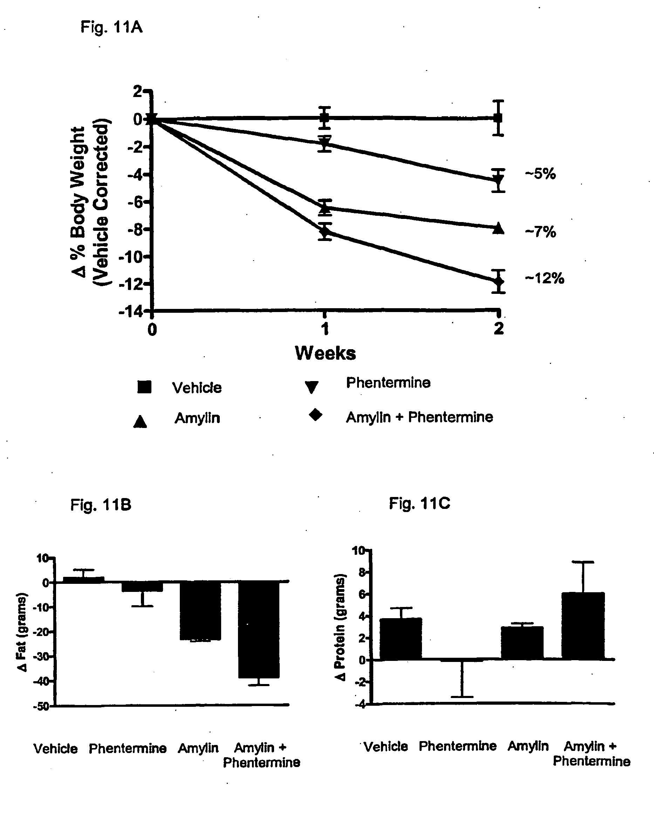

The graph in Figure 11A depicts vehicle-corrected changes in percent body weight of the treatment groups over the two weeks of treatment Phentermine administration alone resulted in about a 5 % decrease in body weight and amylin administration alone resulted in about a 7 % decease in body weight. The percent decrease in body weight in response to administration of a combination of amylin and phentermine was about 12%. Figures 11B and 11C depict changes in body fat and changes in body protein, respectively, produced after the two weeks of treatment. A modest amount of fat mass loss was evident with the treatment of phentermine alone and a greater amount of fat mass loss was evident with the treatment of amylin alone. When amylin and phentermine were administered in combination, a synergistic effect was obtained (Fig. 11B). Lean (protein) mass was unchanged or tended to be lost when phentermine was administered alone. Administration of amylin alone preserved lean (protein) mass and the combination of amylin and phentermine tended to have the greatest increase in lean (protein) mass, even while the animals underwent about 12% loss in body weight (Fig. 11C). These results support a metabolic effect of the combination of agents as well as a weight reducing effect.

### Comparative EXAMPLE 5

To demonstrate effects of the combination of amylin and a CB-1 antagonist on changes in body weight and body composition, in-bred DIO (Levin) rats were obtained from Charles Rivers Labs. These rats were developed by Barry Levin from a line of Crl:CD®(SD)BR rats that are prone to become obese on a diet relatively high in fat and energy. They were housed individually in shoebox cages at 22 C in a 12/12-hour light dark cycle. Rats were maintained *ad-libitum* on a moderately high fat diet (32% kcal from fat; Research Diets D1226B) for approximately 6 weeks prior to drug treatment and throughout the experiment. Prior to drug administration rats had typically attained a mean body weight of 500 g. Rats were habituated to oral gavage for 1 week prior to treatment. Rimonabant was administered at a range of doses (0.1, 0.3,1.0,3.0. 10 mg/kg/day) by oral gavage. Amylin (dissolved in 50% DMSO sterile water) or vehicle was administered by mini-pump (100 µg/kg/day). Rimonabant was always delivered just prior to lights out. Food intake and body weight were measured at 1 and 2 weeks post-treatment. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment. This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment - % body fat at baseline = change in % body fat).

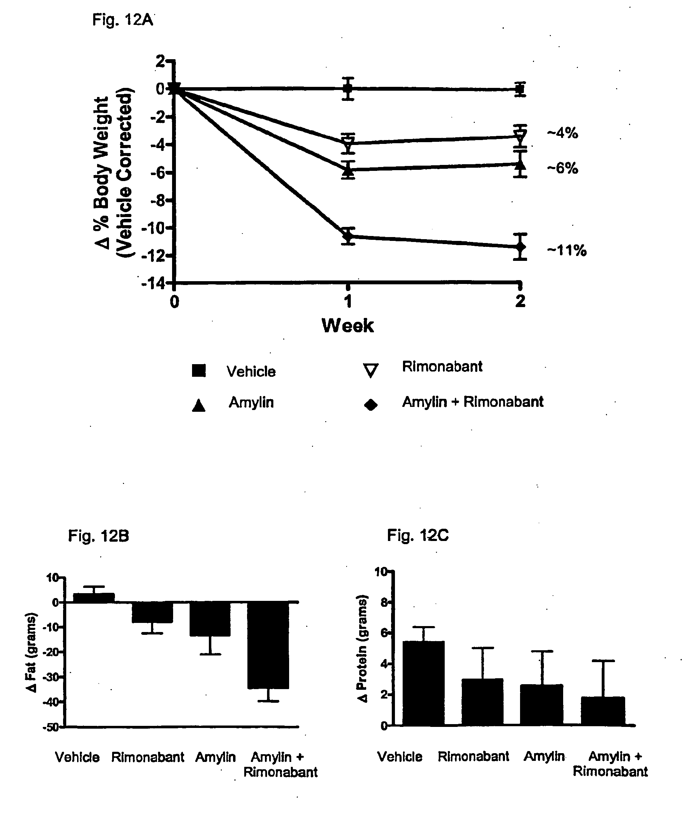

The graph in Figure 12A depicts vehicle-corrected changes in percent body weight of the treatment groups over the two weeks of treatment Rimonabant administration alone resulted in about a 4 % decrease in body weight and amylin administration alone resulted in about a 6% decease in body weight. The percent decrease in body weight in response to administration of a combination of amylin and rimonabant was about 11%. Figures 12B and 12C depict changes in body fat and changes in body protein, respectively, produced after the two weeks of treatment. Fat mass loss was evident with the treatment of either amylin alone or rimonabant alone, and a synergistic effect was obtained when both amylin and rimonabant were administered in combination (Fig. 12B). Administration of amylin alone, rimonabant alone, and amylin + rimonabant in combination resulted in a relatively equivalent increase in lean (protein) mass (Fig. 12C). These results support a metabolic effect of the combination of agents as well as a weight reducing effect.

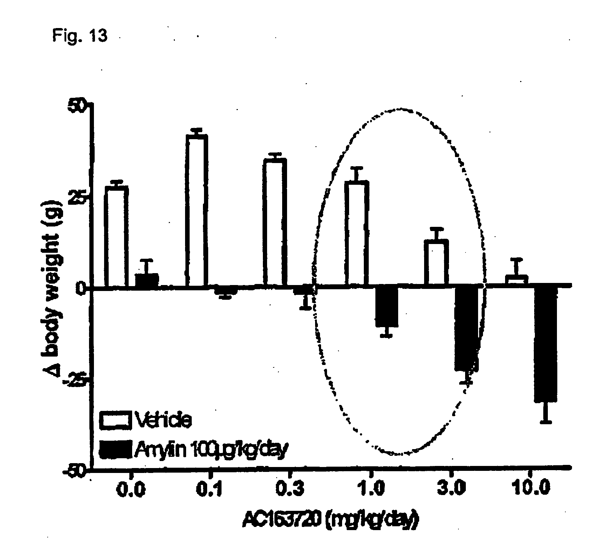

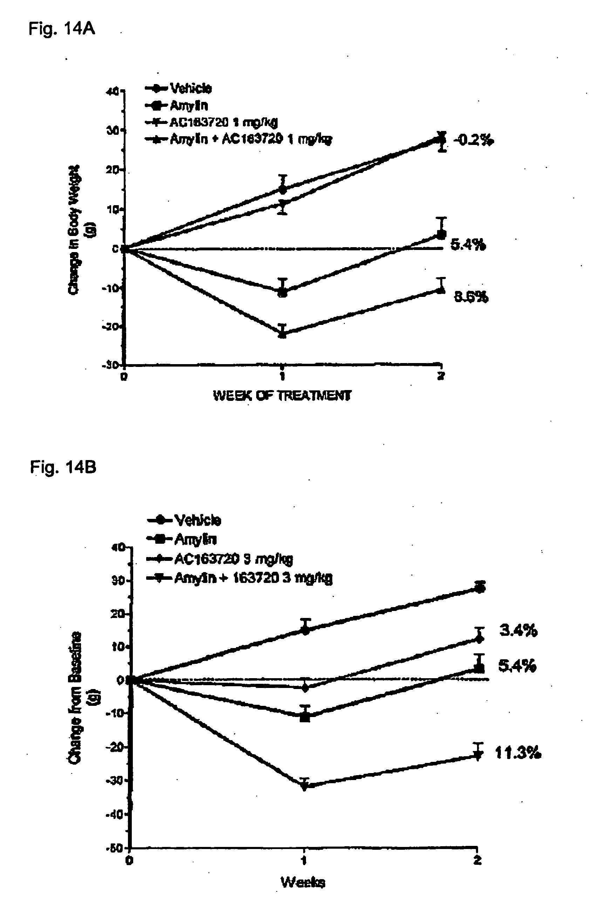

In another assay, the CB-1 antagonist rimonanbant(AC163720) was administered in combination with amylin. DIO prone rats were maintained *ad-libitum* on a moderately high fat diet (32% kcal from fat; Research Diets D1226B) for 6 weeks prior to drug treatment At the end of the fattening period they typically have a mean body weight of 500 g. Rats were then divided into treatment groups and implanted with one subcutaneous mini-pump (Durect Corp) and inserted with an oral gavage. The mini-pump contained either vehicle (50% DMSO in water) or amylin (100 µg/kg/day) while the oral gavage administered either sterile water or a range of doses of rimonabant (AC163720) (0.1, 0.3,1.0, 3.0,10.0 mg/kg/day). Change in body weight after 2 weeks is depicted in Figure 13 and two of these combinations (circled) are highlighted in Figures 14A and 14B in more detail.

### Comparative EXAMPLE 6

To demonstrate effects of the combination of amylin and an exendin analog, ¹⁴Leu-exendin-4: His Gly Glu Gly Thr Phe Thr Ser Asp Leu Ser Lys Gln Leu Glu Glu Glu Ala Val Arg Leu Phe Ile Glu Trp Leu Lys Asn Gly Gly Pro Ser Ser Gly Ala Pro Pro Pro Ser (SEQ ID NO:190), on changes in body weight and body composition, DIO male rats were fattened and divided into four treatment groups, as described above. Prior to drug administration rats had typically attained a mean body weight of 500 g Exendin-4 analog was administered by minipump at a range of doses (0.3,1, 3, 10, 30 µg/kg/day). Amylin (dissolved in 50% DMSO in water) or vehicle was administered by mini-pump (100 µg/kg/day). Body weight and food intake was recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment This enabled the calculation of changes in actual grams of fat and dry lean tissue (e.g., grams of body fat after treatment -grams of body fat at baseline = change in grams of body fat) and changes in % body composition for fat and dry lean tissue (e.g., % body fat after treatment - % body fat at baseline = change in % body fat).

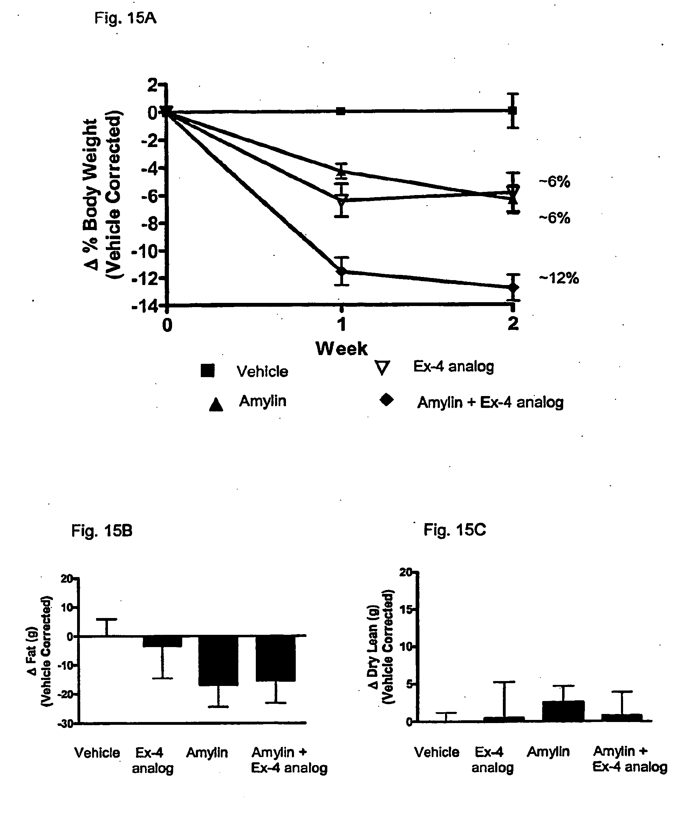

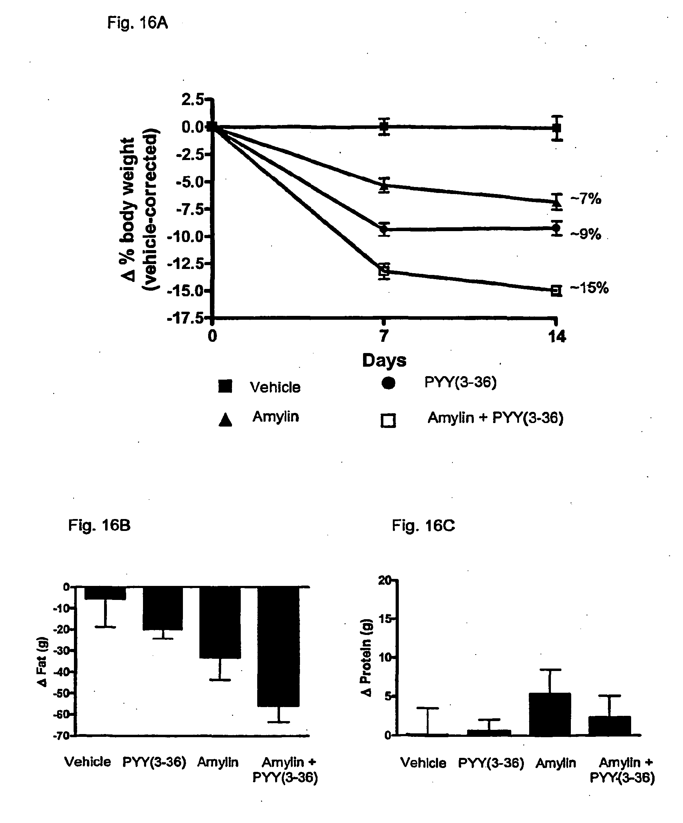

The graph in Figure 15A depicts vehicle-corrected changes in percent body weight of the treatment groups over the two weeks of treatment Exendin-4 analog administration alone and amylin administration alone each resulted in about a 6% decrease in body weight The percent decrease in body weight in response to administration of a combination of amylin and Exendin-4 analog was about 12%. Figures 16B and 15C depict changes in body fat and changes in body protein, respectively, produced after the two weeks of treatment. Fat mass loss was evident with the treatment of Exendin-4 analog alone. Administration of amylin alone and amylin + Exendin-4 analog in combination resulted in a relatively equivalent decrease in fat mass (Fig. 15B). Administration of amylin alone, Exendin-4 analog alone, and amylin + AC3174 in combination resulted in a relatively equivalent increase in lean (protein) mass (Fig. 15C). These results support a metabolic effect of the combination of agents as well as a weight reducing effect.

### Comparative EXAMPLE 7

To demonstrate effects of the combination of amylin and PYY agonists on changes in body weight and body composition, DIO male rats were fattened and divided into four treatment groups, as described above. Each group was implanted with subcutaneous osmotic mini-pumps designed to deliver vehicle, PYY(3-36) (1000 µg/kg/day), amylin (100 µg/kg/day) or PYY(3-36) (1000 µg/kg/day) + amylin (100 µg/kg/day) for a 14 day period. PYY(3-36) was administered by mini-pump at a range of doses (100, 200, 400, 800, 1000 µg/kg/day). Amylin 100 µg/kg/day (dissolved in 50% DMSO sterile water), PYY(3-36) (dissolved in 50% DMSO sterile water) or vehicle was administered by mini-pump. Food intake and body weight was recorded daily. Body composition was measured prior to and after drug treatment using NMR (Echo Medical Systems, Houston, TX). For body composition measurements, rats were briefly placed (∼1 min) in a well-ventilated plexiglass tube that was then inserted into a specialized rodent NMR machine. Rats were scanned prior to pump implantation and on the final day of the experiment This enabled the calculation of changes in actual grams of fat and dry lean tissue (e.g., grams of body fat after treatment-grams of body fat at baseline = change in grams of body fat) and changes in % body composition for fat and dry lean tissue (e.g., % body fat after treatment -% body fat at baseline = change in % body fat).

The graph in Figure 16A depicts vehicle-corrected changes in percent body weight of the treatment groups over the two weeks of treatment PYY(3-36) administration alone resulted in about a 9% decrease in body weight and amylin administration alone resulted in about a 7% decrease in body weight. The percent decrease in body weight in response to administration of a combination of amylin and PYY(3-36) was about 15%. Figures 16B and 16C depict changes in body fat and changes in body protein, respectively, produced after the two weeks of treatment. Increasing amount of fat mass loss was evident with the treatment of PYY(3-36) alone, amylin alone, and amylin + PYY(3-36) in combination (Fig. 16B). Administration of amylin alone and amylin + PYY(3-36) in combination resulted in an increase in lean (protein) mass (Fig. 16C). These results support a metabolic effect of the combination of agents as well as a weight reducing effect.

## Claims

1. Use of a first anti-obesity agent selected from amylin or an amylin agonist having at least 80 % amino acid sequence identity to SEQ ID NO : 1 having amylin activity for the manufacture of a medicament for treating obesity in a human subject, wherein the medicament is to be administered in combination with a second anti-obesity agent selected from a leptin, a leptin derivative or a leptin agonist, wherein the agents are to be administered in amounts effective to reduce the body weight of the subject by at least 10%, and wherein the serum leptin concentration of the subject is greater than 10 ng/mL prior to administration of the anti-obesity agents.

2. Use of claim 1, wherein the anti-obesity agents in combination are to be administered by simultaneous or concurrent administration.

3. Use of claims 1 to 2, wherein the second anti-obesity agent is selected from recombinant human leptin and recombinant human methionyl leptin.

4. Use of any of claims 1 to 3, wherein the anti-obesity agents in combination are to be administered simultaneously.

## Patentansprüche

1. Verwendung eines ersten Agens gegen Obesität, ausgewählt aus Amylin oder einem Amylin-Agonisten mit mindestens 80 % Aminosäuresequenz-Identität mit der SEQ ID NR. 1, die Amylin-Aktivität aufweist, zur Herstellung eines Medikaments zur Behandlung von Obesität bei einem humanen Subjekt, wobei das Medikament in Kombination mit einem zweiten Agens gegen Obesität zu verabreichen ist, welches aus einem Leptin, einem Leptin-Derivat und einem Leptin-Agonisten ausgewählt ist, wobei die Agenzien in Mengen zu verabreichen sind, welche zur Reduzierung des Körpergewichts des Subjekts um mindestens 10 % wirksam sind, und wobei die Serum-Leptin-Konzentration des Subjekts vor der Verabreichung der Agenzien gegen Obesität größer als 10 ng/ml ist.

2. Verwendung nach Anspruch 1, wobei die Agenzien gegen Obesität in Kombination durch simultane oder gleichzeitige Verabreichung zu verabreichen sind.

3. Verwendung nach den Ansprüchen 1 bis 2, wobei das zweite Agens gegen Obesität aus rekombinatem humanen Leptin und rekombinantem humanen Methionyl-Leptin ausgewählt ist.

4. Verwendung nach einem der Ansprüche 1 bis 3, wobei die Agenzien gegen Obesität in Kombination simultan zu verabreichen sind.

## Revendications

1. Utilisation d'un premier agent anti-obésité, choisi parmi l'amyline et un agoniste d'amyline présentant une séquence d'acides aminés identique à un degré d'au moins 80 % à la Séquence N° 1 et doté d'une activité d'amyline, en vue de la fabrication d'un médicament conçu pour traiter une obésité chez un sujet humain, lequel médicament devra être administré en association avec un deuxième agent anti-obésité, choisi parmi la leptine, un dérivé de leptine et un agoniste de leptine, et lesquels agents devront être administrés en des quantités qui ont pour effet de faire diminuer d'au moins 10 % le poids corporel d'un sujet, la concentration de leptine dans le sérum du sujet étant supérieure à 10 ng/mL avant l'administration desdits agents anti-obésité.

2. Utilisation conforme à la revendication 1, les agents anti-obésité en association devant être administrés simultanément ou concomitamment.

3. Utilisation conforme à la revendication 1 à 2, le deuxième agent anti-obésité étant choisi parmi de la leptine humaine recombinante et de la méthionyl-leptine humaine recombinante.

4. Utilisation conforme à l'une des revendications 1 à 3, les agents anti-obésité en association devant être administrés simultanément.
